Europäisches Patentamt

European Patent Office

Office européen des brevets

(19)

(11) **EP 1 116 790 A1**

(12) **EUROPEAN PATENT APPLICATION**

(43) Date of publication:
**18.07.2001 Bulletin 2001/29**

(51) Int Cl.⁷: **C12N 15/12**, C07K 14/475, C12Q 1/68, A61K 48/00, A01K 67/027, G01N 33/68

(21) Application number: **00400100.4**

(22) Date of filing: **14.01.2000**

(84) Designated Contracting States:
**AT BE CH CY DE DK ES FI FR GB GR IE IT LI LU MC NL PT SE**
Designated Extension States:
**AL LT LV MK RO SI**

(71) Applicants:
• **INSTITUT NATIONAL DE LA SANTE ET DE LA RECHERCHE MEDICALE (INSERM)**
  **75654 Paris Cédex 13 (FR)**
• **ASSOCIATION FRANCAISE CONTRE LES MYOPATHIES**
  **F-75013 Paris (FR)**

(72) Inventors:
• **Melki, Judith**
  **75015 Paris (FR)**

• **Dierich, Andrée**
  **67100 Strasbourg (FR)**
• **Cifuentes-Diaz, Carmen**
  **78000 Versailles (FR)**
• **Le Meur, Marianne**
  **67200 Strasbourg (FR)**
• **Frugier, Tony**
  **13120 Gardanne (FR)**
• **Tiziano, Francesco Danilo**
  **00168 Rome (IT)**

(74) Representative: **Desaix, Anne et al**
  **Ernest Gutmann - Yves Plasseraud S.A.**
  **3, rue Chauveau-Lagarde**
  **75008 Paris (FR)**

(54) **Murine models characterized by conditional mutation of SMN gene - use for study of spinal muscular atrophy and other neurodegenerative diseases and muscular myopathies**

(57)  The present application describes non-human mamals, especially mice, which carry a conditional mutation in the survival motor neuron gene (SMN gene), said mutation enabling a tissue-specific expression pattern of a mutated SMN protein. A preferred mutation is in the exon 7 which enables the expression of a SMN protein having a truncated C-terminal end (SMNΔC15). These animals provide models for study of degenerative processes and in particular for the study of spinal muscular atrophy (SMA) or other neurodegenerative diseases including amyotrophic lateral sclerosis (SLA) or muscular myopathies including dystrophies. In addition to the animal models, the application provides means for the design of therapeutic strategies including for the evaluation of substances that may interact with deficiencies related to the mutation of the SMN gene.

FIGURE 1

EP 1 116 790 A1

**Description**

The present application describes animals, especially mice, which carry a conditional mutation of the survival of motor neuron gene *(SMN* gene), said animals providing interesting models for the study of degenerative processes and in particular for the study of Spinal Muscular Atrophy or other neurodegenerative diseases including amyotrophic lateral sclerosis (SLA) or muscular myopathies including dystrophies.

In addition to the animal models, the invention provides means for the design of therapeutic strategies including for the evaluation of substances that may interact with deficiencies related to the mutation of the *SMN* gene. Said means include the obtained animals in accordance with the invention, or products derived therefrom, especially cell cultures or, more generally, any product, including genes or proteins, that may be identified through the use of the animals of the invention.

Spinal Muscular Atrophy (SMA) is a neuromuscular disorder characterized by degeneration of anterior horn cells of the spinal cord, leading to muscle denervation responsible for symmetrical limb and trunk paralysis associated with muscle atrophy. SMA represents the second most common fatal recessive autosomal disorder after cystic fibrosis (Roberts et al., 1970; Pearn, 1973, 1978; Czeizel and Hamula, 1989). Based on the age of onset and clinical course, childhood SMA is subdivided into three clinical forms (Munsat, 1991). The most severe form, type I or Werdnig Hoffmann disease, is characterized by generalized muscle weakness leading to severe hypotonia either at birth or within the first six months of age, and with death usually occuring within the first two years of age. The intermediate form, type II, is characterized by a later onset. Patients presenting with this form are able to sit but not to walk unaided and survive beyond 4 years. In type III SMA, proximal muscle weakness occurs after 18 months of age. A positional cloning strategy allowed the identification of the survival of motor neuron gene (*SMN*) as the SMA-determining gene (Melki et al., 1994; Lefebvre et al., 1995). The *SMN* gene has been also described in European patent application EP 0708178 published on April 26, 1996. *SMN* exists in duplicate, with only five nucleotides distinguishing it from its highly homologous copy gene, *SMNc*. One of these nucleotides is located in exon 7 and is responsible for the alternative splicing of exon 7 that is specific to the *SMNc* transcripts (Lefebvre et al., 1995; Burglen et al., 1996; Lorson et al., 1999). In SMA, 95% of patients carry homozygous deletion or conversion events of the *SMN* exon 7 and the remaining 5% carry intragenic mutations within the *SMN* gene (reviewed in Melki, 1997).

*SMN* encodes a protein of 294 amino acids (SMN) which locates both to the cytoplasm and to a nuclear structure called « gem », for gemini of coiled bodies (Liu and Dreyfuss, 1996). SMN forms part of a large protein complex that contains the SMN-interacting protein 1 (SIP1) and Sm proteins of the spliceosomal uridine-rich small ribonucleoproteins (U snRNPs, Liu et al., 1997; Fischer et al., 1997). The N-terminal domain of SMN (amino acids 1-90) has been implicated in SIP1 binding and association with RNA, whereas the domain comprising amino acids 249-278 has been shown to be important for SMN oligomerization and Sm protein binding (Liu et al., 1997; Lorson et al., 1998). Therefore, it appears that SMN plays a key role in spliceosomal biogenesis by bringing together the components of the spliceosomal complex (Pellizzoni et al., 1998). Protein analysis in SMA patients indicated that both the SMN and *SMNc* genes are translated into proteins of similar molecular weight (Lefebvre et al., 1997; Coovert et al., 1997). A strong correlation between the amount of the protein encoded by the *SMNc* gene and the clinical severity of the disease has been demonstrated in SMA patients indicating that *SMNc* remains functional in patients and can be regarded as a modifier gene in SMA (Lefebvre et al., 1997). Neither homozygous deletion of both the SMN and *SMNc* genes nor the complete absence of the SMN protein have been reported to date, suggesting that this likely results in an extremely severe form of SMA and possibly generates a non viable fetus.

The murine homolog of the human *SMN* gene has been mapped, cloned and sequenced; it shares a high degree of homology with its human counterpart (Viollet et al., 1997; DiDonato et al., 1997). In contrast to the human, the mouse *SMN* gene is not duplicated, which may explain the fact that the conventional knockout of the *SMN* gene results in embryonic lethality (Viollet et al., 1997; Schrank et al., 1997, Li Hsiu Mei Hsieh et al (January 2000), Nature Genetics, vol. 24).

The molecular mechanisms by which the deletion or conversion of *SMN* exon 7 encoding the C-terminal end of the protein (amino acids 279-294) lead to motor neuron degeneration remain unclear (Lewin, 1995).

To gain insight into the pathogenesis of SMA and to understand the function of SMN inferred from the SMA phenotype, the inventors have carried out a conditional deletion of the murine *SMN* exon 7, the most frequent mutation found in SMA patients. In particular, starting from a Cre/loxP recombination system of bacteriophage P1 (Sternberg and Hamilton, 1981; Sauer and Henderson, 1988, Gu et al., 1994), they have designed and characterized animal models that present interesting genotypic or phenotypic characteristics.

Among the animals which have been obtained by the inventors and which harbour a conditional mutation of the *SMN* gene, some of them are particularly interesting as they appear to be beneficial as a model of the human SMA. Other animals have been obtained that can be used as models for the study of muscular myopathies. Animals have been obtained according to the invention, which carry the proposed conditional mutation of the *SMN* gene, that provide for the first time, information that may be relevant for the identification of the targets of the deficient *SMN* gene in SMA or in other degenerative pathologies.

The invention provides non-human mammals carrying a conditional mutation in the *SMN* gene, said mutation enabling a tissue-specific expression pattern of a mutated SMN protein.

By "conditional mutation", it is intended a mutation which expression on one or both alleles of the *SMN* gene, is obtained under the control of a determined factor, and for instance is dependent upon the expression of a given DNA sequence, or upon the expression of a protein in the animal, or alternatively upon any appropriate substance, which would be administered to the animal in conditions enabling the sought mutation to occur. Said factor involved in the occurrence of the conditional mutagenesis, directly or indirectly interacts on one allele of the *SMN* gene in the animal, to enable its mutation to take place. Said interaction may be conditional in time with respect to the expression of the *SMN* gene and or may be conditional in space.

For instance, said mutation can be expressed, as a tissue-specific mutation.

In a particular embodiment of the present invention, a conditional mutation of a gene, especially of the *SMN* gene encompasses in a first aspect, the case where the finally obtained animal carries a homozygous mutation of its endogeneous *SMN* gene, said mutation only occurring in controlled conditions especially as a tissue-specific homozygous mutation. According to another embodiment it also relates to the case where the obtained animal exhibits a heterozygous mutation that may become homozygous under the control of a molecule (protein, gene...) capable of transforming the heterozygous mutation in a homozygous mutation of the gene, said homozygous mutation possibly expressing in a subsequent offspring of the animals presenting the heterozygous mutation, in specific determined conditions such as tissue-specific expression.

Particular embodiments describing how a conditional mutation can be achieved are disclosed in the following pages and in the examples below.

In particular, the invention is directed to non-human mammals which carry a conditional mutation in the *SMN* gene, which is obtained only in determined tissues and provide a specific expression pattern of the mutated gene, such as a mutated SMN protein.

According to a preferred embodiment of the invention, the non-human mammal which is defined above carries a conditional mutation in the *SMN* gene, which is a mutation of Exon 7 of said gene, that enables the expression of a SMN protein having a truncated C-terminal end.

Said truncated C-terminal protein has been described as the protein which is expressed in Human suffering from SMA, as a consequence of a mutation of the *SMN* gene and of an alternative splicing of RNA transcribed from the *SMNc* copy of the *SMN* gene in human.

One particular truncated protein is the SMN protein lacking the 15 C-terminal amino acid residues (SMNAC15).

Advantageously, in accordance with the invention, the above-defined mutation in the *SMN* gene is a deletion of exon 7.

In mice, where no copy of the *SMN* gene is present, the expression of the *SMN* gene devoid of exon 7 in accordance with the conditions defined herewith, provide an animal model that reproduces the substantial characteristics of the phenotype linked to SMA in Human.

The mutated non-human mammal according to the invention, which replies to one or several of the above given definitions, can be obtained by crossing animals harbouring the genetic constructs that are necessary to obtain the homozygous mutation of the *SMN* gene. The invention also relates to the animals which are the offspring of the above defined non-human mammals.

In order to achieve the conditional mutation, site specific recombination systems permitting homologous recombination to occur under the control of proteins especially enzymes, expressed conditionally, may be used.

The invention provides in a preferred embodiment, non-human mammal carrying a conditional mutation in the *SMN* gene, wherein one allele of said gene is recombined in order to replace the exon 7 by a heterologous sequence comprising the exon 7, said exon 7 being flanked by loxP sequences (thereby producing a "floxed exon 7"). The insertion of the sequence comprising exon 7 flanked by loxP sequences can be obtained by homologous recombination.

Such loxP sequences are used as a part of the Cre/loxP recombination system, the Cre recombinase enbling the conditionally deletion of the sequence inserted between the loxP sites.

Therefore, when the Cre-recombinase is expressed in cells containing the mutated floxed allele of the *SMN* gene, comprising the floxed exon 7 sequence, the exon 7 is deleted.

This allows the achievement of the conditional mutation. Furthermore, this mutation can be restricted to specific tissues or cells, when the gene coding for Cre recombinase is placed under the control of regulation sequences, including promoter sequences, presenting a tissue specificity.

Any functionally equivalent allowing mutagenesis in a conditional way, may be used in accordance with the present invention. In particular, one may cite as an example of such mutagenesis system, including homologous recombination systems, yeast FLP/FRT system Cre-ERT system (Metzger D. et al, 1995, PNAS 92, p.6991-6995).

A particularly preferred non-human mammal according to the invention is an animal which is obtained by crossing an animal which carries a homozygous mutated *SMN* gene (*SMN^{F7/F7}*), thereby carrying both alleles having a floxed exon 7 (F7), with an animal carrying the Cre coding sequence under the control of tissue-specific regulation sequences and one *SMN* allele having a deleted exon 7 (Δ7).

A preferred non-human mammals according to one or several including combined definitions given above, encompasses a non-human mammal wherein one allele of the *SMN* gene carries a floxed *SMN* exon 7 (SMN$^{F7}$) the other allele having a deletion of exon 7, said non-human mammal further carrying a sequence encoding the Cre-recombinase under the control of tissue specific regulation sequences. Said non-human mammal is characterized by the genotype: *(SMN $^{F7/Δ7}$, -Cre+)*. Various appropriate promoters are disclosed below.

The preparation of such a mutated animal is described in details in the examples below.

Advantageously, the inventors have observed that the *SMN* gene carrying on one allele the floxed exon 7 and on the other allele a deletion of exon 7, provides better controlled conditional mutation in mice obtained after crossing said (*SMN$^{F7/F7}$*) mice with mice expressing the Cre gene and which carries a deleted SMN exon 7, preferentially in a tissue-specific manner. Alternatively the invention however also relates to the use of (*SMN$^{F7/F7}$*) mice for crossing with mice expressing the Cre coding sequence and carrying the SMN F7 allele.

In a particular embodiment of the invention, the mutation of the *SMN* gene is intended to replace only exon 7 or a part of said exon sufficient to express a mutated SMN protein by a heterologous sequence. However, where appropriate, the endogeneous *SMN* gene of the animal may be replaced by a corresponding sequence obtained from an animal of a different species or from a Human. Such corresponding sequence is designated by the expression heterologous sequence.

The invention also relates to non-human mammals according to one or several, including any possible combination, of the above given definitions, wherein the mutation of the *SMN* gene is a conditional homozygous mutation, which expression is restricted to a neuronal tissue.

According to this embodiment, the conditional mutation becomes homozygous as a result of the activity of the site-specific recombination system, and is restricted to a specific tissue, when said recombination system is placed under the control of tissue-specific regulation sequences. Such tissue-specific regulation sequences encompass the promoter of the Neuron Specific Enolase gene (NSE).

The non-human mammals according to the invention are advantageously animals wherein the conditional mutation of the *SMN* gene is a homozygous mutation which expression is restricted to neuronal tissues including the spinal cord or cerebelum. In particular, said mutation is directed to motor neurons.

According to another embodiment of the invention, the non-human mammal is characterized in that the mutation of the *SMN* gene is a conditional homozygous mutation which expression is restricted to the skeletal muscle tissue.

Particular mammals according to the invention include rodent, especially mice, or other animals from canine species, rabbits, non human primates bovines etc.

Particularly preferred animals are mice, which exhibit the following genotype: *(SMN $^{F7/Δ7}$, NSE-Cre+)*.

These mice are capable of expressing in neuronal tissues preferentially, a mutated *SMN* gene which has been deleted on both alleles for the exon 7.

The NSE sequence is a sequence of the promoter of the neuron specific enolase gene, which enables the neuron specific expression of the Cre recombinase. Therefore, such mice are expressing a mutated SMN protein in neurons, including especially in motor neurons and is protected from lethality that would occur as a result of a homozygous mutation of the *SMN* gene in all the cells of the animal.

Mice having the above-said genotype, are capable of expressing a SMA phenotype, thereby providing a model of interest for the study of the human SMA.

The invention also relates to mice which exhibit the following genotype: (SMN$^{F7/Δ7}$, HC79-Cre+). These mice wherein the Cre gene is placed under the control of a promoter sequence which is tissue specific for the expression of the Cre recombinase in skeletal muscle.

In addition, the invention encompasses the use of synthetic promoters, or ubiquitary promoters when they are inducible (i.e., CMV promoter induced by the interferon).

Mice having this genotype are of particular interest since they exhibit a muscular distrophic phenotype. Indeed, the inventors have shown that surprisingly abnormal expression of *SMN* transcript in skeletal muscle is associated with muscular dystrophy, providing data that suggest that SMN protein may have a critical role in development or differenciation of skeletal muscle during post natal period.

Said mice expressing a muscular specific mutation of the *SMN* gene can also provide interesting models for the study of muscular myoopathies.

Mice having the *SMN $^{F7/Δ7}$, HC79-Cre+* genotype have allowed the inventors to study the expression of the gene encoding dystrophin, revealing lower expression of the protein encoded by said gene, dystrophin, or its complete lack at the muscle membrane.

Thus, the invention provides means that enable the study of neurodegenerative processes in animal models of human SMA using a neuronal promoter and further provides means that enable to study the impact of a conditional, muscle specific mutation of the *SMN* gene in muscular dystrophy using a muscular promoter, thereby offering an animal model for the study of therapeutic approaches in SMA and more generally in various muscular dystrophies. This includes the screening of drug candidates or more generally of molecules that can interact with deficiencies happening as a consequence of the mutation of the *SMN* gene or which interact with target genes of the *SMN* gene.

In order to obtain the conditional mutation of the invention, expressed in a tissue specific manner, especially in neuronal tissue or in muscular tissue, various promoters can be proposed.

Other neuronal promoters may be used including the promoters of the following genes have been already characterized and can be used to drive Cre recombinase expression in neuronal tissues: Neurofilament, synapsin I, Islet I, pcp-2, clamodulin III, dopamine B-hydroxylase, nitric oxide synthetase, choline acetyl transferase tyrosine hydroxylase, PDGF B chain.

Other skeletal muscle promoters including the promoters of the following genes have been already characterized and can be used to drive Cre recombinase expression in muscle tissues: acetylcholine receptor alpha 2 subunit, myogenin, aldolase A, creatine kinase.

The inventors have characterized the non-human mammals obtained according to the invention, and have shown that they exhibit particular morphological properties that confirm their interest in providing animal models for the study of SMA or of other neurodegenerative disorders or muscular myopathies.

Accordingly, mice have been shown to present the following properties:

1) they express a truncated SMN protein in motor neurons said protein being located to the cytoplasm of cells but not to the nuclear structure of said cells.
2) they exhibit abnormal coiled bodies and it is essentially devoid of gem structures in motor neurons.
3) they do not show a substantial loss in neurons.

These properties have been observed in patients suffering from SMA, except for the absence of substantial loss in neurons which absence of loss apparently accompanies the initial stages of the disease.

The absence of abnormal coiled bodies in human may be observed as a result of the expression of a residual coiled bodies.

In addition, the inventors have shown that mice carrying a conditional homozygous mutation of the *SMN* gene in motor neurons exhibit morphological characteristics:

1) morphological abnormalities in skeletal muscles including denervation of skeletal muscle fibers,
2) morphological abnormalities in spinal cord, including morphological changes in nuclei of motor neurons, in the absence of significant loss of motor neurons.

The absence of significant loss of motor neurons suggests that this phenomenum is a late manifestation of the disease, providing the possibility to prevent neuronal death in the human disease.

The invention also relates to cells derived from the above-defined non-human mammals and especially to cells derived from neuronal populations derived from animal having the genotype (SMN$^{F7/\Delta7}$, NSE-Cre) or any animal expressing the mutation in neuronal tissue specifically, or from skeletal muscular tissue derived from animal having the genotype (SMN$^{F7/\Delta7}$, HC79-Cre) or any animal expressing the mutation in muscular tissue specifically. The cells can be either primary cultures of neurons or muscular cells. It can also be immortalized cells.

Cells derived from the above non-human mammals can also be embryonic stem cells that may be used in replacement of the above animals for the study of degenerative processes accompanying mutation of the *SMN* gene. Such population of cells may also be used for the design of therapeutic approaches, including for the screening of molecules which would be of interest for a therapeutic approach. The expression "therapeutic" encompasses every investigation used in experimental, preclinical or clinical strategies for the design of drugs for the treatment of SMA or for the treatment of disorders associated with the mutation of the *SMN* gene. In particular, the invention relates to the design of therapeutic approaches and the identification through the use of the above-defined non-human mammals and population of cells, of compounds including genes or proteins, or any substance capable of stabilizing, improving or even curing one or several defects due to the mutation of the *SMN* gene.

Thus, the invention provides a process for the comparative analysis of transcripts, present in first and second populations of cells of spinal cord, wherein said first population of cells exhibits a homozygous mutation of the *SMN* gene thereby expressing in said first population of cells, a SMN protein which is truncated at its C-terminal end and wherein, said second population of cells of spinal cord exhibits a normal *SMN* gene thereby expressing a normal SMN protein, said process comprising:

-   isolating the RNA transcripts of said first and second populations of cells of spinal cord, to thereby obtain respectively a first and a second populations of transcripts,

- detecting the specific presence or absence, in said first or second population of transcripts, of one of several specific transcripts, thereby detecting differentially expressed transcripts in said one or second population of transcripts.

The above designated spinal cord tissue is obtained from the above described animals which carry the conditional mutation in neuronal tissues.

Alternatively the tested population of cells is derived from muscular tissue, especially from skeletal muscular tissue, obtained from animals expressing the mutation of the invention in muscular tissue.

Several specific methods useful for the implementation of the above-disclosed process are described in the Examples which follow.

The invention encompasses applying the above defined process in any tissue where the presence or the absence of a gene or transcript of interest may be analyzed.

Said process may further comprise the isolation and identification of the RNA transcripts which are specific to only one of the compared first and second populations of transcripts ( designated differentially expressed transcripts).

If a differentially expressed RNA transcript is identified by the above-defined process, said transcript can be advantageously retro-transcribed and thereby, provide the corresponding DNA sequence for further study including for use of the design of a therapeutic approach.

In a further step, said process can advantageously comprise the expression, for instance in determined cells, of the product encoded by one or several of the differentially expressed transcripts which have been identified.

According to a particular embodiment of the invention, the process can be carried out after contacting and administering a determined molecule to the population of cells which is tested for differential expression of transcripts.

The invention also relates to a process for the comparative analysis of the protein expression pattern in a first and a second populations of cells of spinal cord, wherein said first population of cells exhibits a homozygous mutation of the *SMN* gene leading to the expression in said first population of cells, of a SMN protein which is truncated at its C-terminal end and wherein, said second population of cells of spinal cord exhibits a normal *SMN* gene thereby expressing a normal SMN protein in said second population of cells, said process comprising:

- comparing the protein expression pattern of said first and second populations of cells of spinal cord,
- detecting the presence or absence, in said first and second populations of cells, of one of several specific proteins that is differentially expressed in one of said both populations of cells.

Alternatively the tested population of cells is derived from muscular tissue, especially from skeletal muscular tissue, derived from an animal of the invention that expresses the conditional mutation specifically in the muscular tissue.

As defined for the process for the comparative analysis of the transcript expression pattern, the process for the comparative analysis of the protein expression pattern can be implemented after the administration of the determined molecule to the assay population of cells. Such a molecule can be chosen among those which have been described above in relation with the comparative method for the analysis of the expression pattern of transcripts. It can be administered directly to the animal before extracting the proteins to be compared. Alternatively, it can be administered to a population of cells derived from the animals, if said population of cells is used further for the extraction of the proteins to be analyzed.

The invention also relates to a process for the evaluation of the effect of a molecule or of an intact *SMN* gene on the of neurodegenerative process occurring in SMA disease, and especially on the possible reversion of the enurodegenerative process, which comprises:

- - administering to a non-human mammal defined above or to a culture of cells defined above, an intact *SMN* gene placed under the control of regulatory sequences enabling the conditional spatial and/or temporal expression of said intact *SMN* gene in said non-human mammal or said culture of cells,
- detecting a change in the phenotype or the restoration of a normal phenotype in said non-human mammal or said culture of cells.

The intact *SMN* gene can be delivered by any appropriate vector including but not limited to recombinant vectors selected from viral vectors, retroviral vectors, lentiviral vectors, human or non-human, especially canine adenovirus vectors, or a synthetic vectors.

According to another embodiment, the intact *SMN* gene can be administered under the form of a fusion protein, for example as a fusion with the tat sequence of HIV retrovirus.

Alternatively, this intact gene may be delivered by encapsulated or non-encapsulated cells from the animals of the invention, or from other organisms.

The molecule can be any molecule defined for the use of the various embodiments of the invention. It can be administered according to various protocols some of them being illustrated in the examples.

The process for the evaluation of possible reversion of the neurodegenerative process occurring in SMA disease, can also be carried out in conditions where the administration of the *SMN* gene is accompanied by the previous, simultaneous or delayed administration of a determined molecule capable of interacting with or enhancing the activity of the intact *SMN* gene.

Molecules, genes or generally substances which may be used in various screening or study processes of the invention as defined above, can include molecules selected from: neurotrophic factors, neuroprotective agents, neuromodulators, neurotransmitter molecules, neuropeptides, the SMN protein, differentially expressed genes or protein products identified in a process described above.

Particular uses of the non-human mammals are of the cell populations derived therefrom in accordance with the invention, may comprise the study of splicing defect of RNAs in cells, including splicing defects of RNA that may be considered as a target for the *SMN* gene.

Another particular interest of the invention is the use of the above non-human models or populations of cells derived therefrom, for the analysis of the transcription and/or RNA processing of the dystrophin gene. More generally, the result obtained by the inventors which show that the mutation of the *SMN* gene gives rise to an abnormal expression pattern of the dystrophin gene has led the inventors to propose that the *SMN* gene may be involved in the transcription process of maturation of genes having large intronic sequences.

Genes with large intronic sequences include dystrophin, amyloid precursor protein (APP), human retinoblastoma susceptibility gene or human breakpoint cluster region (BCR) gene.

Especially, the *SMN* gene could have an activity on the processing of genes having large intronic sequences, normally expressed in neurons and muscles.

As a consequence other tissues than those cited above can be analysed for the detection of the expression or lack of expression of the gene of interest, depending upon the location of said gene. Thus the invention encompasses for example the analysis of brain, liver, cardiac tissues in addition to the above cited tissues. In particular tissues where the Cre recombinase, or the factor controlling the conditional mutation, is expressed can be analysed and compared to tissues where the enzyme is not expressed.

The invention also relates to a process for the evaluation of the activity of a determined molecule on neurodegenerative processes of neuronal or muscle populations, comprising administering said molecule to one of the above non-human mammals or to a population of cells derived from said non human mammal and further detecting the effect of said molecule on the tested cells.

In a preferred embodiment of the invention, these cells are motor neurons.

The invention also concerns the specific DNA constructs which are described in the examples below, for the preparation of non-human mammal defined in accordance with the invention. The primer sequences which are disclosed in the following examples are especially comprised within the scope of the invention.

The invention is further illustrated by the following examples and by the drawings which are described below.

**Legend to Figures**

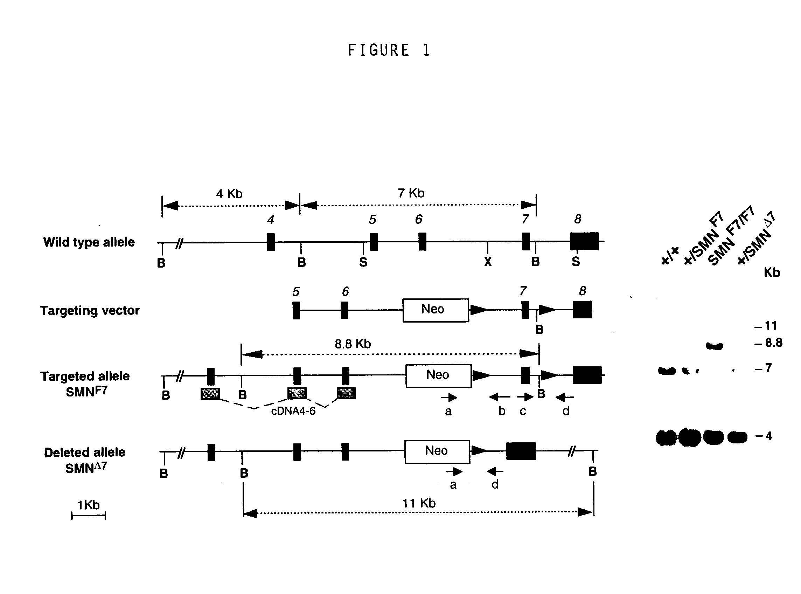

**Figure 1. Conditional mutagenesis of mouse *SMN* exon 7.**

The left panel shows the wild type genomic *SMN* locus surrounding the targeted exon, the targeting vector, the loxP-flanked *SMN* exon 7 allele (*SMN^{F7}*) and the Cre-mediated deleted allele (*SMN^{Δ7}*). Filled rectangles indicate exons and filled arrows indicate the position of loxP sequences. The locations of the PCR primers used to screen for homologous recombination or Cre-mediated deletion and the cDNA probe used to confirm this are also given. Shown in the right panel is Southern blot analysis of DNA from tail biopsies of wild type (+/+), (*SMN^{F7/+}*), (*SMN^{F7/F7}*) and (*SMN^{Δ7/+}*) mice. Digestion with BamHI generates a 7 kb wild-type fragment, an 8.8 kb « floxed » fragment and a 11 kb deleted fragment which are detected with a probe derived from *SMN* cDNA and containing exons 4 to 6. Note that the insertion of the *NeoR* cassette into intron 6 leads to an increase in size of the BamHI restriction fragment encompassing the loxP-flanked *SMN* locus (8.8 kb). Cre-mediated deletion of the DNA fragment between the two loxP sites leads to the loss of the BamHI site located in intron 7 and thus resulting in an increase of the restriction fragment encompassing the deleted allele (11 kb). An additional fragment of 4 kb corresponding to a more upstream restriction fragment is detected in wild type, « floxed » and deleted alleles using the same probe. Abbreviations: B, BamHI; X, Xhol; S, Sall; NeoR, neomycine resistance cassette gene; Primers (a), *PHR*5; (b), XG3; (c), *ex7sou1;* (d), GS8.

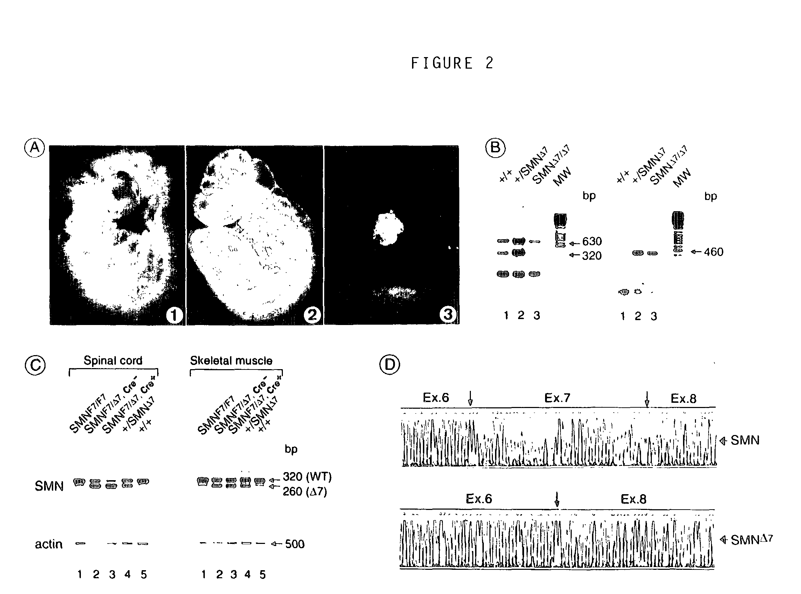

**Figure 2. Deletion of *SMN* exon 7 in all cell types.**

(A) Nine day post-coitum embryos belonging to the same litter of (*SMN^{Δ7/+}*) intercrosses are shown at the

same magnification. ($SMN^{\Delta7/+}$, 2) embryo is indistinguishable from wild type (+/+, 1), while the ($SMN^{\Delta7/\Delta7}$, 3) embryo is completely resorbed. (B) Genotype analysis of DNA extracted from the corresponding yolk sacs using PCR amplification. Left panel: primers *int7b* and *ex7sou2* located upstream of exon 7 and in exon 7 respectively, were used to amplify the wild type fragment (320 bp) which is detected in (+/+), and ($SMN^{\Delta 7/+}$) but not in ($SMN^{\Delta7/\Delta7}$) embryos. PCR amplification using primers flanking *SMN* exon 4 was used as internal positive control (630 bp). Right panel: detection of the $SMN^{\Delta7}$ allele by PCR amplification using primers *pHR5* and GS8 generating a 460 bp fragment derived from the deleted allele. The PCR product is only detectable following excision of SMN exon 7 by the Cre recombinase. The expected 460 bp fragment was successfully amplified in ($SMN^{\Delta7/+}$) and ($SMN^{\Delta7/\Delta7}$) but not in (+/+) mice. (C) Transcript analysis of *SMN* from spinal cord or skeletal muscle tissues derived from wild type and mutant mice. RT-PCR amplification of *SMN* was performed on total RNA using primers flanking *SMN* exon 7. RT-PCR of α-actin transcripts was used as an internal control. RT-PCR products were separated by agarose gel electrophoresis and labeled with ethidium bromide. RT-PCR amplification reveals a single product of 320 bp in tissues from (+/+, WT) and ($SMN^{F7/F7}$) mice indicating the absence of an alternative splicing of exon 7 in *SMN* transcripts in mice carrying these genotypes. In addition to the full length RT-PCR product, a shorter product of 260bp is detected in ($SMN^{\Delta7/+}$), ($SMN^{F7/\Delta7}$, Cre-) and ($SMN^{F7/\Delta7}$, Cre+) mice. Note the slight increase of the truncated transcript in both spinal cord and skeletal muscle of ($SMN^{F7/\Delta7}$, Cre-) mice when compared with the RNA pattern of ($SMN^{\Delta7/+}$) mice. A marked reduction in the amount of full-length *SMN* transcript is detected in spinal cord but not in skeletal muscle of ($SMN^{F7/\Delta7}$, Cre+) mice compared with that observed in mice harbouring the other genotypes. (D) Sequence analysis of the RT-PCR products generated from RNA of ($SMN^{\Delta7/+}$) mice. Full length and truncated *SMN* transcripts were separated on an 8% polyacrylamide gel, excised and reamplified using the same primers prior to direct sequencing. Arrows indicate the boundaries of each exon.

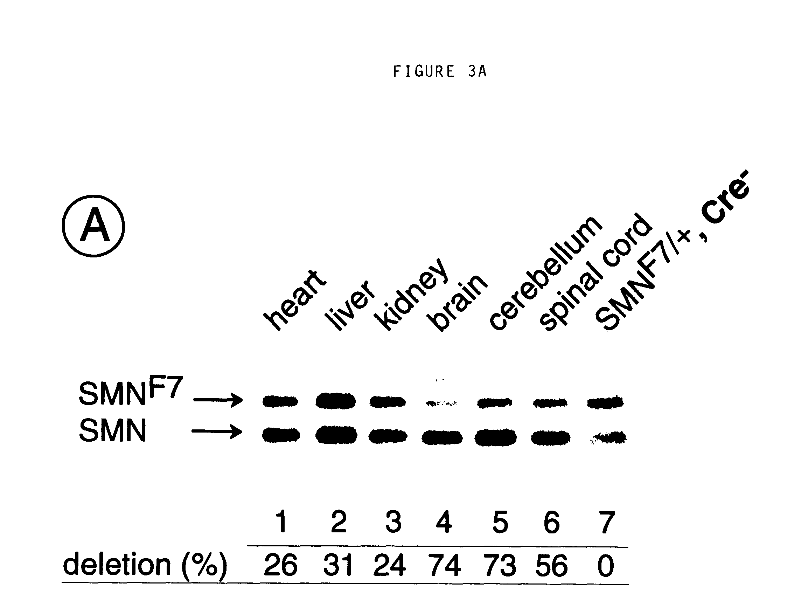

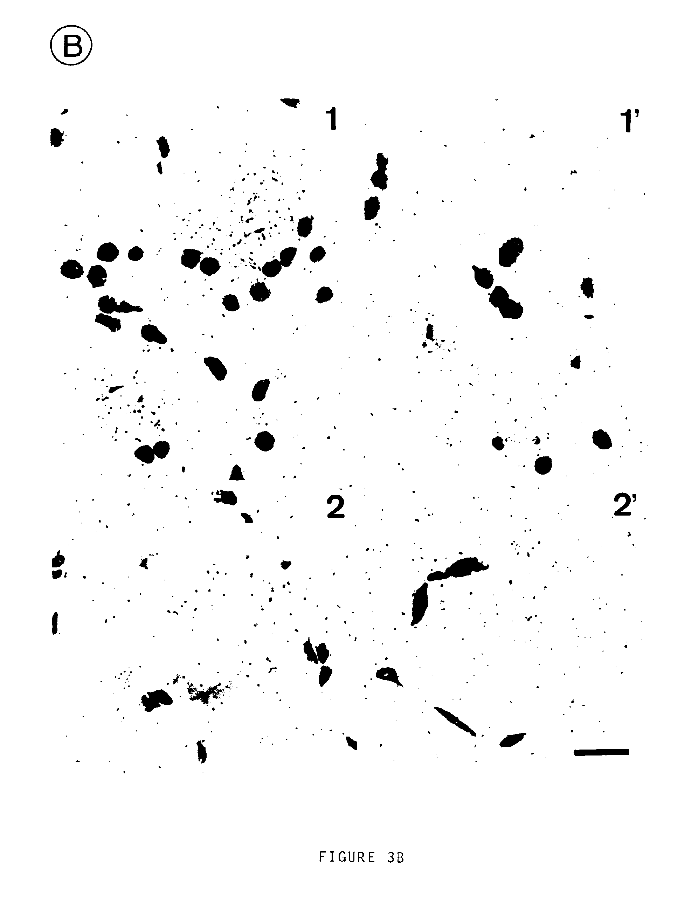

**Figure 3. Characterization of Cre recombinase activity of the NSE39-Cre transgenic line**

(A) To test the efficiency of DNA excision by the Cre recombinase, NSE39-Cre transgenic mice were crossed with mice carrying the $SMN^{F7}$ allele and double transgenic mice were selected. The wild type and $SMN^{F7}$ alleles were simultaneously amplified by PCR using *ex7sou1* and GS8 primers. The efficiency of Cre-mediated deletion was deduced from the ratio of $SMN^{F7}$ to wild type band intensity in various adult tissues of the double transgenic mouse (lanes 1 to 6) compared with the ratio in $SMN^{F7/+}$ mouse lacking Cre transgene (lane 7). *(B)In situ* hybridization using a Cre recombinase riboprobe was performed on spinal cord transverse sections counterstained with toluidine blue. Antisense riboprobe reveals an expression pattern restricted to neurons in the adult spinal cord (1) while no hybridization signal is detectable using the sense riboprobe. Scale bar: 10 μm

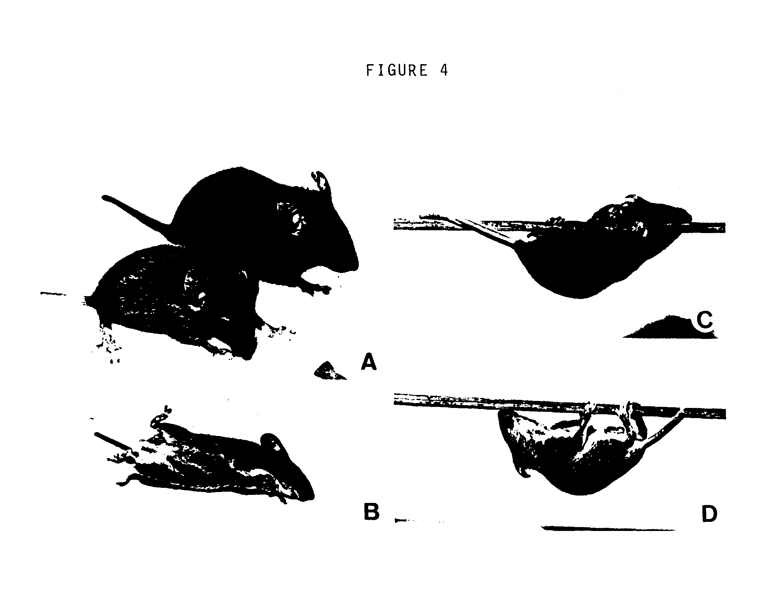

**Figure 4. Motor defects in *(SMN^{F7/\Delta7}, Cre+)* mice.**

(A) Note the abnormal posture of the hindlimbs and the smaller size of the 3 week-old *($SMN^{F7/\Delta7}$, Cre+)* mouse (agouti color) when compared to the wild type mouse (black color). The mutant mouse has a severe impairment in its ability to right itself when intentionally inverted (B) and displays severe hypotonia characterized by a defect of flexor muscles of the limbs and neck when suspended on a horizontal thread (D) compared with the posture from the wild type animal (C).

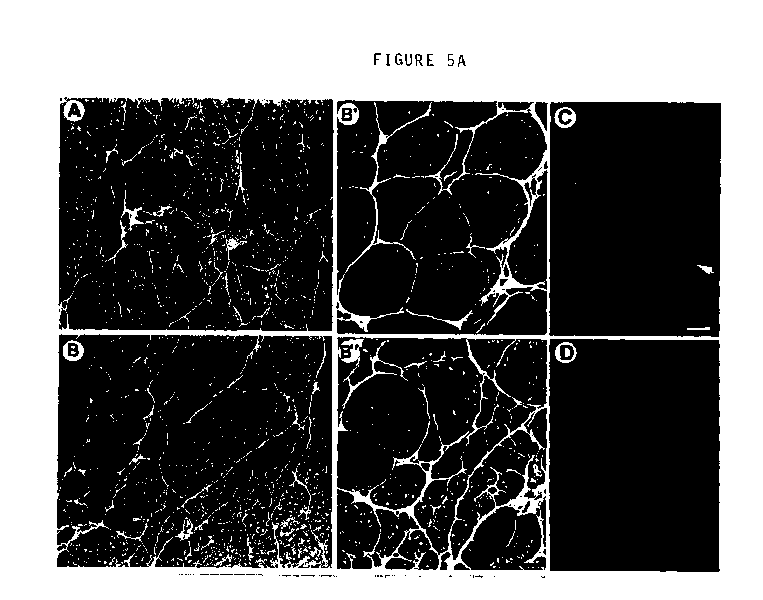

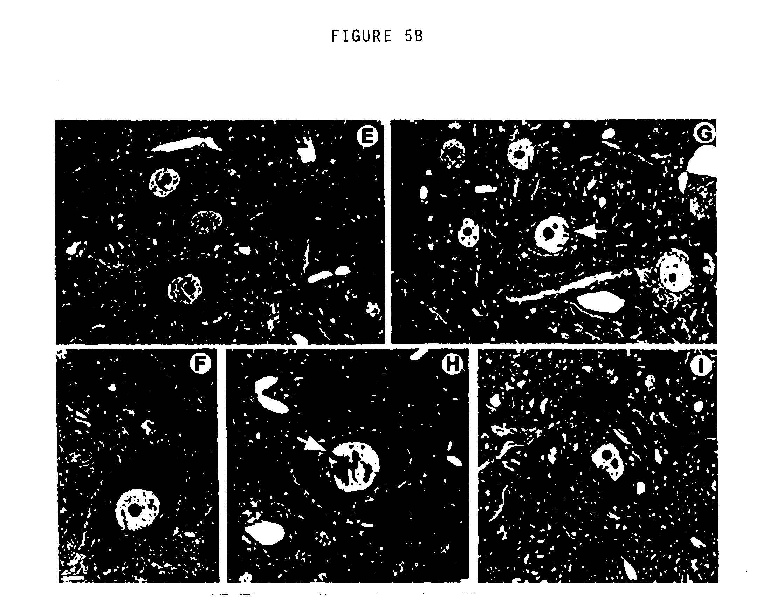

**Figure 5. Skeletal muscle morphology of wild type and *(SMN^{F7/\Delta7}, Cre+)* mice.**

(A-B) Hematoxylin and eosin staining of transverse sections of gastrocnemius muscle from 2 week-old mice. (B,B') Groups of atrophic muscle fibers are evident in the mutant mouse compared with the diameter of muscle fibers of control muscle (A). (B,B') In neighbouring areas, angular fibers (indicated by a triangle) are intermixed with normal-appearing fibers. Neithe mecrosis, inflammatory cell infiltration nor fibrosis is observed. Scale bar: 20μm (C-D) Labelling of the acetylcholine receptors using rhodamine-conjugated α-bungarotoxin (AchR molecular probes Eugene, OR, dilution, 1:10000) on transverse sections of gastrocnemius muscle of 2 week-old wild type and *($SMN^{F7/\Delta7}$, Cre+)* mice. (C) In the control mouse, AChRs are concentrated at the neuromuscular junction with their characteristic α-bungarotoxin curved staining (arrow). (D) In *($SMN^{F7/\Delta7}$, Cre+)* mice, a marked extrajunctional labelling of AChRs was observed in a pattern of expression characteristic for skeletal muscle denervation. Scale bar: 10 μm (C and D).(E and F) In control mice, motor neurons are large cells of the anterior horns that contain abundant cytoplasm and a prominent nucleololus in a large rounded nucleus. (G-I) In $SMN^{F7/\Delta7}$, *Cre+* mice, note the abnormal morphology of the nuclei, which are characterized by the presence of indentations in the nuclear membrane of motor neurons. These indentations appear as intensely blue staining regions within the nuclei at the nuclear-cytoplasmic boundary (arrows). These abnormal nuclei are observed at different levels of the spinal cord including at the cervical, thoracic and lumbar levels. Note the granular aspect of motor neuron cytoplasm in (I). Scale bar: 4μm.

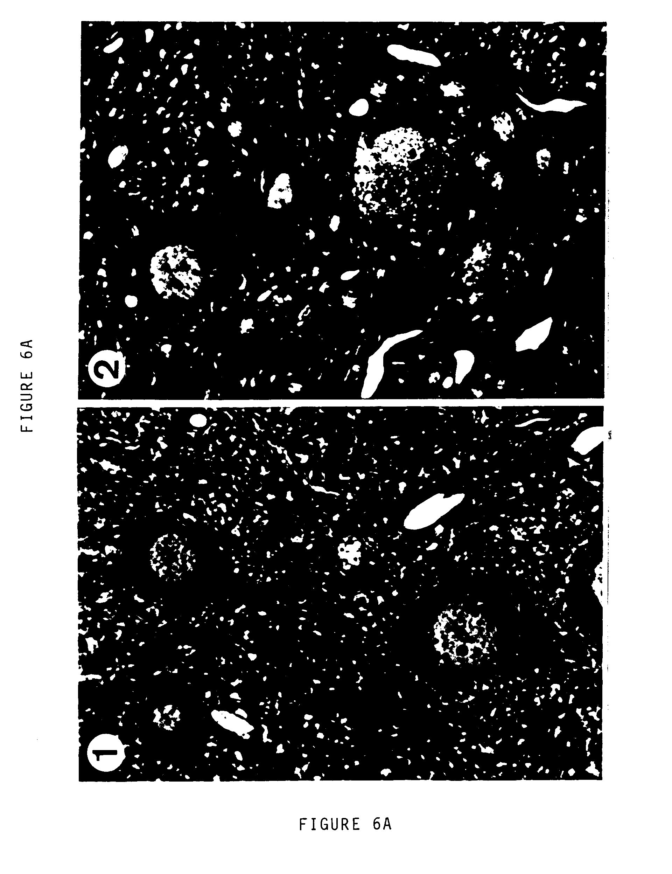

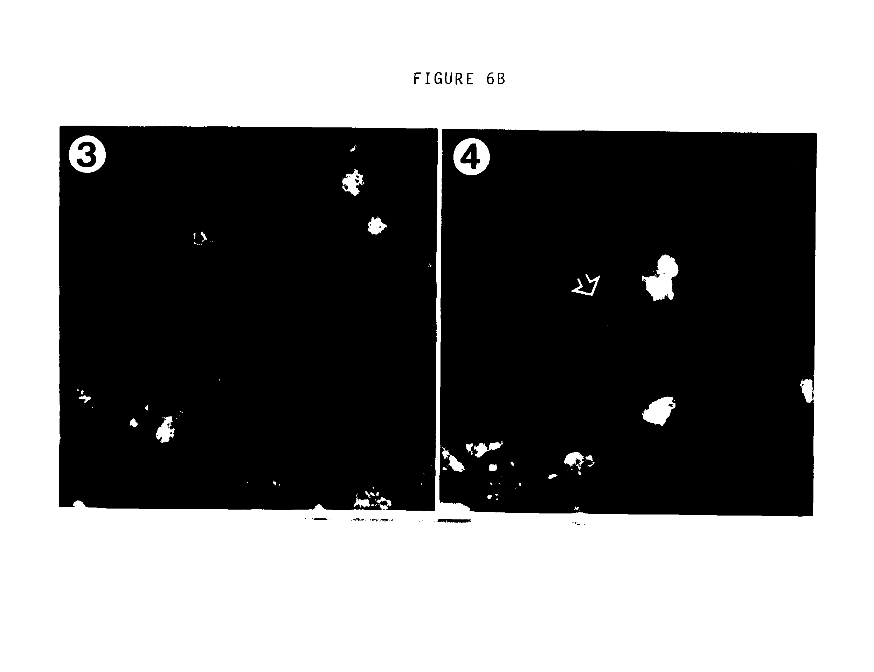

**Figure 6. Spinal cord morphology of wild type and *(SMN^{F7/Δ7}, Cre+)* mice.**

Toluidine blue staining of transverse semithin sections (1μm) of spinal cord from 2 week-old mice. (A and B) In control mice, motor neurons are large cells of the anterior horns that contain abundant cytoplasm and a prominent nucleolus in a large rounded nucleus. (C, D and E) In *(SMN^{F7/Δ7}, Cre+)* mice, note the abnormal morphology of the nuclei, which are characterized by the presence of indentations in the nuclear membrane of motor neurons. These indentations appear as intensely blue staining regions within the nuclei at the nuclear-cytoplasmic boundary (arrow). Note the granular aspect of motor neuron cytoplasm in E. Scale bar: 4 μm.

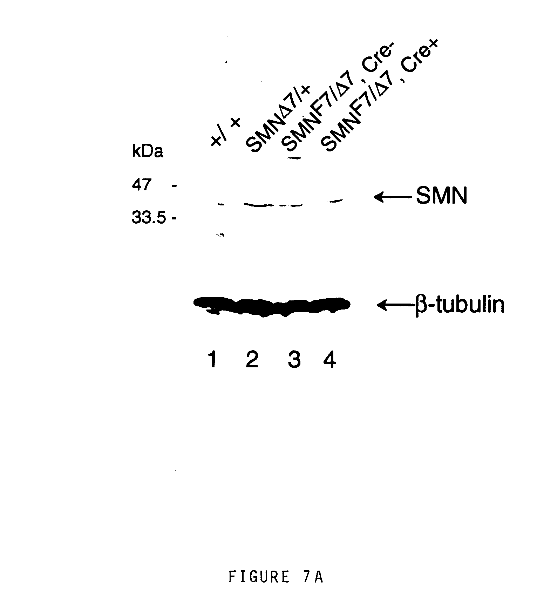

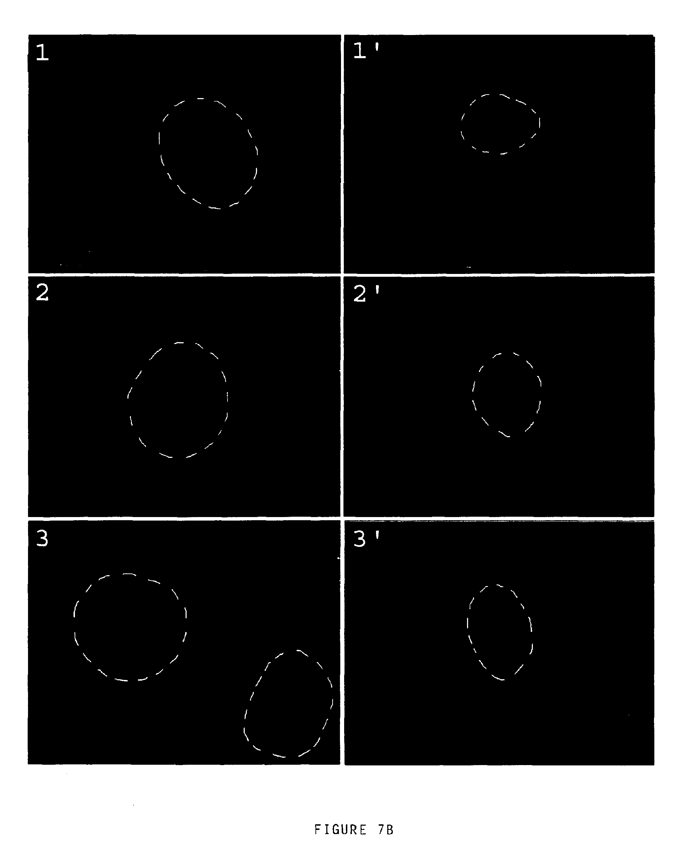

**Figure 7. Immunostaining of SMN, SIP1 or coilin.**

(A) Immunoblot analysis of total protein extracts from the spinal cord of wild type and mutant mice. The membranes were incubated with anti-SMN antibody (1:2000) and anti β-tubulin monoclonal antibody (5 :5000 dilution Amersham) as an intermal control. The membranes were washed and incubated with anti-mouse IgG conjugated to horseradish peroxydase and the immune complexes were revealed using chemiluminescent detection reagents (Pierce).
(B) Confocal laser microscopy analysis of SMN (1,1'), SIP1 (2,2') and coilin (3,3') on transverse spinal cord sections from wild type (1,2,3) and mutant (*SMN_N^{F7/Δ7}, Cre+*) mice (1',2',3'). (1) In the control mouse, the 2B1 anti-SMN antibody stains both the cytoplasm and the gems in the nuclei of motor neurons (arrow) while it fails to detect the nuclear staining of SMN-containing gems in *(SMN^{F7/Δ7}, Cre+)* mice. Note the presence of aggregates in the cytoplasm (indicated by short arrow) (1'). (2) In the control, immunohistochemistry with the anti-SIP1 antibody (2[E]17) shows that SIP1 is concentrated in gems as previously described in HeLa cells (arrows). (2') In *(SMN^{F7/Δ7}, Cre+)* mice, note the absence of gems in motor neurons. (3) In the control, the Anti-p80 coilin (204,10) stains several coiled bodies in the nuclei of motor neurons (arrow), while it stains large nuclear aggregates in motor neurons of *(SMN^{F7/Δ7}, Cre+)* (3' arrow). Scale bar: 4 pm

I GENERATION OF MICE AFFECTED WITH SPINAL MUSCULAR ATROPHY (SMA) PHENOTYPE.

**Experimental procedures**

**Gene targeting and generation of loxP-flanked *SMN* exon 7 mice**

A 6 kb DNA fragment containing exons 5 to 8 of the murine *SMN* gene was amplified by long-range PCR from 129Sv total genomic DNA using primers located in exons 5 and 8 *(ex5sou2,* 5'- TGC TGG ATG CCC CCG TTC CCT TCA- 3' and *ex8sou,* 5'- GGC ACG CTC TGC TGC TGA CTT AG -3', respectively, Viollet et al., 1997). A total of 100 ng of genomic DNA was used for PCR amplification in a buffer containing: 500 μM each dNTP, 300 nM of each primer, 2.2 mM MgCI2, 50 mM Tris-HCl pH 9.2, 16 mM (NH4)2S04 and 1 unit of Taq DNA and of Pwo polymerase (Boehringer). PCR was performed in a Perkin Elmer GenAmp 9600 thermal cycler with the following conditions: 10 cycles of denaturation for 10 sec at 93°C and annealing-extension for 15 min at 65°C followed by 20 cycles of denaturation for 10 sec at 93°C and annealing-extension at 65°C for 15 min with an elongation of 20 sec for each cycle. A detailed restriction map of the PCR amplification product was identical to that expected from analysis of total genomic DNA. Following a fill-in reaction, the PCR product was cloned into the Smal restriction site of pUC18. Sequence analysis of each exon and intron-exon junction was performed and no nucleotide discrepancy was found from that previously described (Viollet et al., 1997). A BamHl restriction fragment containing the region from exon 5 to the 5' end of intron 7 was subcloned into pUC18 to generate the plasmid 58P8, and a BamHI-filled EcoRI restriction fragment containing the region from the 3' end of intron 7 to exon 8 was subcloned into pUC18 to generate the plasmid BBP10. A Scal-Smal restriction fragment derived from PHR68 (derived from PHR55, Schwartz F et al A dominant positive and negative selectable gene for use in mammalian cells. PNAS USA 1991 88:10416-20) and containing a loxP site was introduced into the seventh intron downstream of the BamHl restriction site of the 58P8 plasmid to generate the plasmid 58L11. A filled BamHI-Sacl restriction fragment of BB131 was inserted into the Smal-Sacl restriction site of 58L11 giving the plasmid B2. Finally, a cassette containing a neomycin resistance gene *(NeoR)* driven by the phosphoglycerate kinase *(PGK)* promoter and followed by a loxP site was introduced into the Xhol site of the sixth intron to generate the plasmid BEF2 (figure 1). Sequence analysis confirmed that both loxP sites were in the same orientation. The final targeting construct contains 3 kb of flanking genomic sequence further upstream of the neomycin cassette and a 1 kb fragment downstream of the second loxP site (figure 1). To generate homologous recombinants, P1 embryonic stem cells (ES) derived from 129Sv mice (10[7] cells) were transfected with 10 μg of the linearized targeting construct by electroporation. The transfected ES cells were selected in 150 μg/ml of G418. Resistant colonies were trypsinized individually and expanded in a 48-well plate for genotyping. Homologous recombinant clones were selected using both PCR amplification and Southern blot analyses. ES clones containing the *NeoR* cassette were first selected by PCR using primers XG3 (5'- CTG GCC CAA ATC ACA ACA TAA -3') and *PHR5* (5'-TTC TCT TGA TTC CCA CTT TGT GGT TC-3') located in the mouse genomic sequence downstream of the *NeoR* cassette and in the *NeoR* cassette, respectively. Positive clones were then further characterized by Southern blotting using probes corresponding to cDNA fragments containing exons 4 to 6 (termed probe *cDNA4-6), SMN* exon 8 or the internal *NeoR* gene to identify homologous recombinant clones. Targeted clones were injected into blastocysts which were transferred into pseudopregnant foster mothers. Chimeras identified by the presence of an agouti coat colour were test-mated with C57BL/6J females. Agouti offspring were tested for the presence of the loxP-flanked *SMN* exon 7 allele (*SMN$^{F7}$*) by both Southern blot analysis of DNA from tail biopsy using *cDNA4-6* probe and by PCR amplification as described above.

## Plasmid construction, generation of Cre recombinase transgenic line and quantification of Cre-mediated deletion

A 2100 bp EcoRl/Hindill restriction fragment of the *pNSE-CAT* plasmid, containing the rat neuron specific enolase gene promoter *(NSE,* Forss-Petter et al., 1990), was cloned into the pGS5 plasmid (Miniou P et al Gene targeting restricted to mouse striated muscle lineage Nucl. Acid Res. 1999, 27:27-31) which contains the Cre recombinase gene separated from the enolase promoter by the rabbit α-globin intron and followed by the *SV40* polyadenylation signal. Subsequently, the purified Notl fragment was microinjected into the pronuclei of fertilized eggs from (C57BL/6J X SJL) F1 mice and implanted in pseudopregnant foster mothers. The parental transmission of the transgene was confirmed both by PCR amplification and Southern blotting. Primers Cre1 (5'-CCG GTC GAT GCA ACG AGT GAT-3') and Cre2 (5'- ACC AGA GTC ATC CTT AGC GCC-3') were used to amplify a 790 bp fragment of the Cre recombinase transgene. This PCR product was used as a probe for Southern blot analyses of DNA extracted from tail biopsy.
To test the efficiency of DNA excision by the Cre recombinase, transgenic mice were crossed with mice carrying the *SMN$^{F7}$* allele and double transgenic mice were selected. Genomic DNA was extracted from a variety of adult tissues. The wild type and *SMN$^{F7}$* alleles were simultaneously amplified by PCR using one set of primers *(ex7sou1-GS8,* see genotype analysis section). The efficiency of excision was estimated by comparing the intensity of the band amplified from the *SMN$^{F7}$* allele with that of the band amplified from the wild type allele which differs only by the absence of the loxP site. After 15 cycles, PCR products were separated by agarose gel electrophoresis, transferred to Nylon-N+ membrane and hybridized using the wild type PCR product as a probe. Intensity of bands was quantified using the Biorad GS700 densitometer and Molecular Analyst software. *In situ* hybridization using a Cre recombinase riboprobe was performed on cryosections of directly frozen tissues as previously described (Niederreither and Dollé, 1998). A BamHl-Xhol restriction fragment of the *Cre* recombinase gene derived from the pGS5 plasmid was subcloned into pBluescript plasmid KS(-). After digestion with either BamHl or Xhol restriction enzymes, sense and antisense RNA synthesis was performed using T3 or T7 RNA polymerase and labeled with $^{35}$S-CTP. Slides were counterstained with toluidine blue.

## Genotype analysis

To genotype adult mice, DNA was extracted from tail biopsy or tissues following lysis (Tris HCl pH 8, 100 mM, EDTA 5 mM, SDS 0.2%, NaCl 200 mM, proteinase K 200 µg/ml overnight at 55°C), phenol extraction and ethanol precipitation. Two microliters (200 ng) of the above preparation were used for PCR amplification in buffer containing 200 µM each dNTP, 1 µM of each primer, 1.5 mM MgCl2, 10 mM Tris HCl pH 8.3 and 50 mM KCl. For genotyping embryos, yolk sacs were digested in 200 µl lysis buffer (KCl 50 mM, Tris HCl pH 8.3, 10 mM, MgCl2 1.5 mM, Nonidet P40 0.45%, Tween 20 0.45% and 100 µg/ml of proteinase K) overnight at 55°C. Two microliters of this preparation were used for PCR amplification after denaturation of proteinase K. Primers *int7b* (5'-CTC ACA GAG ATC CTC TTG CCC TGA-3') located upstream of exon 7 and *ex7sou2* (5'-AAT TTG TAT GTG AGC ACT TTC CTT CT-3') located in exon 7 were used to amplify a wild type fragment. Primers *Ex7sou1* (5'-AGA AGG AAA GTG CTC ACA TAC AAA TT-3') and *GS8* (5'-TGT CTA TAA TCC TCA TGC TAT GGA G-3') located in exon 7 and in the seventh intron downstream of the loxP sequence, respectively, were used to amplify either the wild type allele (435 bp), the *SMN$^{F7}$* allele (635 bp) or both. PCR amplification of *SMN* exon 4 using primers flanking this exon *(Sin41RB,* 5'- GAA GCC AGC TTA TAA GAG CAG CTC-3', and *Mint3B,* 5'-CCG AAA GAT GTC CAT TCC TGA AAG) was used as an internal positive control. Detection of Cre-mediated deletion of *SMN* exon 7 was based on both Southern blot analysis using probe *cDNA4-6* and PCR amplification using primers *PHR5* and *GS8* generating a fragment of 460 bp derived from the deleted allele. For Southern blot analysis, 8 to 10 pg of total genomic DNA were digested with BamHl and transferred to a Hybond-N$^+$ membrane (Amersham) for probing following standard procedures.

## RT-PCR amplification and sequence analyses

Total RNA was extracted from freshly isolated tissues using the Trizol procedure (Gibco-BRL). RT-PCR amplification was performed using specific primers flanking *SMN* exon 7 and located in exon 6 *(ex6.5,* 5'- ATA ATC CCG CCA CCC CCT-3') and 8 *(ex8sou).* Alpha-actin transcripts were used as an internal control and amplified with primers *actin2* (5'- GAG CCA CCG ATC CAC ACT GAG TAC-3') and *actin3* (5'- TCT GGA CCT GGC CGG TCG CGA CCT-3'). RT-PCR products were analyzed following agarose gel electrophoresis. For sequence analysis, the RT-PCR products were separated on an 8% polyacrylamide gel, excised and reamplified using the same primers. Purified PCR products were then directly sequenced using the dideoxynucleotide chain termination method and analyzed on an ABI model 373A DNA automated sequencer.

**Morphological and immunofluorescence analyses**

For toluidine blue staining of the spinal cord, the mice were anesthetized with nembutal and perfused via 0.9% NaCI and 2.5% glutaraldehyde, 0.5% paraformaldehyde in 0.1 M phosphate buffer (PB, pH 7.4). Tissue samples were dissected and immersed in the same fixative for 2 hours, rinsed in PB, and postfixed in 2% osmium tetroxide. After three washes with PBS, each sample was dehydrated in a graded series of ethanol and embedded in Epon. Semithin transverse sections of spinal cord (1 pm) were stained with toluidine blue and examined under a Zeiss Axiophot microscope. For immunostaining of choline acetyl transferase (ChAT), anesthetized mice were perfused with 0.9% NaCI and 4% paraformaldehyde in PB. Tissue samples were rinsed in PB and immersed overnight at 4°C in 15% sucrose in PB. They were then frozen in isopentane pre-cooled in liquid nitrogen. Cryostat sections of 10 μm were incubated with anti-goat anti-ChAT purchased from CHEMICON, CA at a 1:800 dilution, revealed by Cy3-conjugated anti-goat anti-IgG (1/500, Jackson Laboratories) then counterstained with DAPI. Motor neurons were counted when large DAPI stained nuclei associated ChAT labelling of the cytoplasm were observed. Immunostaining of SMN, SIP-1 or coilin was performed on tissues fixed with 4% paraformaldehyde in PB, washed twice with PBS and then in PBS-0.03% Triton X-100 (SMN) PBS- 0.5% Triton X-100 (SIP-1) or PBS-1% Triton X-100 (coilin) for 10 min. Tissue sections were incubated with either monoclonal antibody anti-SMN (2B1, Liu and Dreyfuss, 1996, kindly provided by G. Dreyfuss), anti-SIP1 (2E17, Liu et al., 1997) at a 1/500 dilution or rabbit polyclonal anti-p80 coilin (204,10, Andrade et al., 1991, kindly provided by J. Sleeman) at a 1/500 dilution and revealed by Cy3-conjugated anti- mouse or rabbit IgG at a 1/500 dilution (Jackson Laboratories) then counterstained with DAPI. Confocal laser scanning microscopy analysis was performed using a Sarastro 2000 laser confocal system (Molecular Dynamics, Sunnyvale, CA) mounted on a Nikon Optiphot-2 upright microscope. Series of optical sections were taken at 0.2- 0.3 μm steps.

Histology of skeletal muscle was performed on unfixed samples frozen in isopentane pre-cooled in liquid nitrogen and transverse sections of 8-10 pm thick were stained with hematoxylin and eosin. To identify neuromuscular junctions, acetylcholine receptors (AChR) were labeled with rhodamine-conjugated α-bungarotoxin (Molecular probes Eugene, OR) at a dilution of 1:10000 on unfixed transverse sections of skeletal muscle.

**Western blot analysis**

Tissues were dissected and kept frozen at -80°C. Total proteins were prepared using solutions supplemented with protease inhibitors (2 mM phenylmethylsulfonyl fluoride, 3 μg/ml each pepstatin and anti-papain, 15 μg/ml benzaminidine and 40 μg/ml leupeptin). Spinal cord samples were homogenized with ice-cold buffer consisting of 100 mM Tris-HCI pH 7.5, 1% Triton X-100, 100 mM NaCl, 5 mM EDTA, 0.5 mM $MgCl_2$ and 1 mM β-mercaptoethanol. The protein concentration was determined using the Bradford assay (BioRad Laboratories). The resulting homogenates were diluted in Laemmli sample buffer, boiled for 5 min and either stored at -80°C or processed immediately for gel electrophoresis. The samples were electrophoresed on 12% SDS-polyacrylamide gel and electrotransferred to nitrocellulose membrane (Schleicher and Schuel). The membranes were blocked in 5% non-fat dry milk in PBS 1X for 2 hours at room temperature and consecutively incubated with monoclonal antibody 2B1 (1:2000 dilution) and anti-β-tubulin monoclonal antibody (1:5000 dilution, Amersham) as an internal control. The membranes were washed for 5 min in four changes of PBS-0.05% Tween 20 and incubated at room temperature for 1 h with anti-mouse IgG conjugated to either horseradish peroxydase or alkaline phosphatase and the immune complexes were revealed using chemiluminescent detection reagents (Tropix and Pierce).

**Results**

**Generation of mice carrying a loxP-flanked *SMN* exon 7 allele**

To achieve the conditional mutagenesis of the mouse *SMN* gene, we used a long-range PCR amplification product generated from genomic DNA of the *SMN* exons 5 to 8 to construct a targeting vector that includes two loxP sites flanking the *SMN* exon 7 and a *NeoR* gene (figure 1). The targeting construct was electroporated into mouse embryonic stem cells (ES) and 406 G418-resistant ES colonies were screened by PCR amplification using a neo-specific primer *(PHR5)* and a primer flanking the insertion site of *NeoR* (XG3). Positive clones were further characterized by Southern blotting. Southern blot analysis of genomic DNA following digestion with BamHI was used to identify a 7 kb wild-type fragment and the 8.8 kb loxP-flanked *SMN* exon 7 fragment, when *SMN* cDNA from exon 4 to 6 was used as a probe (figure 1). The membranes were rehybridized with a *NeoR* probe or with *SMN* exon 8 to verify a single integration or a homologous recombination event, respectively. Of 406 G418-resistant ES cells, two clones containing the specifically targeted loxP-flanked *SMN* exon 7 allele ($SMN^{F7}$) through homologous recombination were injected into blastocysts. One clone gave high percentage chimeras. Chimeras were bred to C57BL/6J females and mice harbouring the targeted allele were identified following genotype analysis of agouti offspring. Heterozygous $SMN^{F7}$ mice were crossed, and a strain homozygous for the $SMN^{F7}$ allele was established. $SMN^{F7/F7}$ mice appear similar in size and morphology to both heterozygous and wild type mice. At the RNA level, no alternative splicing of SMN exon 7 was detected by RT-PCR amplification analysis of RNA extracted from various tissues of the $SMN^{F7/F7}$ mice, indicating that neither the *NeoR* gene nor the loxP sites have a deleterious effect on *SMN* splicing (figure 2).

**Deletion of *SMN* exon 7 in all cell types results in early embryonic lethality**

To delete the *SMN* exon 7 in all cell types, mice heterozygous for the $SMN^{F7}$ allele were crossed with a strain carrying the *Cre* recombinase transgene placed under the control of the CMV promoter (termed *CMV-Cre,* Dupe et al., 1997). Cre recombinase was shown previously to be expressed in various tissues, including in the germline. Offspring carrying both the $SMN^{F7}$ allele and *CMV-Cre* transgene were identified and crossed with C57BL/6J mice. A heterozygous deletion of the *SMN* exon 7 ($SMN^{\Delta7}$) was detected in one of two offspring lacking the *CMV-Cre* transgene, indicating that an efficient Cre recombinase activity was present in the germline of the double transgenic progenitor and excluding the possibility of somatic events in offspring (figure 1). $SMN^{\Delta7/+}$ mice gained weight normally after birth and have remained indistinguishable from wild type littermates to date (12 months of age). Histological examination of skeletal muscle did not reveal any morphological changes. To test whether the Cre-mediated deletion of the *SMN* exon 7 resulted in *SMN* transcripts lacking exon 7 as expected, RT-PCR amplification was performed on RNA extracted from various tissues including the spinal cord and skeletal muscle of ($SMN^{\Delta7/+}$) or (+/+) mice using primers flanking *SMN* exon 7 (figure 2). The expected 320 bp product corresponding to the full length *SMN* transcript was generated from RNA extracted from either ($SMN^{\Delta 7/+}$) or (+/+) mice. In the heterozygous $SMN^{\Delta 7/+}$ mice, an additional smaller product of 260 bp was generated as expected, presumably due to the absence of exon 7 (figure 2). To confirm this, the shorter PCR product was sequenced and compared with the wild type product. Sequence analysis revealed that in these transcripts, the Cre-mediated deletion of *SMN* exon 7 gene leads to the generation of transcripts lacking exon 7, exons 6 and 8 being fused resulting in a putative protein with a different C-terminal end (figure 2).

To determine whether the homozygous deletion of *SMN* exon 7 in all cell types would lead to embryonic lethality, as previously described in *SMN* knock out experiment (Schrank et al., 1997) or in a milder phenotype, ($SMN^{\Delta7/+}$) mice were intercrossed. These crosses generated no live $SMN^{\Delta7/\Delta7}$ mice out of 50 newborns analyzed. In addition, the ratio of heterozygous to wild type mice was approximately 2:1, which indicated that $SMN^{\Delta7/\Delta7}$ embryos die *in utero* (table). To determine the timing of the $SMN^{\Delta7/\Delta7}$ lethality, embryos were analyzed during gestation. At 9 days post-coitum, a total of 53 embryos, including 13 with almost complete resorbtion was analyzed (figure 2). Both the morphologically normal and the resorbed embryos were genotyped by PCR amplification using primers *PHR5-GS8* and *int7b-ex7sou2* to detect the deleted and wild type alleles respectively. Normal embryos showed the expected ratio for genotypes (+/+) or ($SMN^{\Delta7/+}$), while all resorbed embryos were homozygous for the $SMN^{\Delta7}$ mutation (figure 2 and table). These data indicate that homozygous deletion of *SMN* exon 7 in all cell types results in embryonic lethality during early development.

| Genotype | (+/+) | (+/SMN$^{\Delta 7}$) | (SMN$^{\Delta 7/\Delta 7}$) | $\chi 2$ |
|---|---|---|---|---|
| Adults (n= 50) | 17 (34%) | 33 (66%) | 0 | 16.68 (p<0.001) |
| 9 d.p.c. embryos (n= 53) | 15 (28.3%) | 25 (47.2%) | 13 (24.5%) | 0.32 (p>0.8) |

Table. SMN gene analysis in adult and 9 day post coitum embryos of (+/SMN$^{\Delta 7}$) mouse intercrosses.

**Conditional homozygous deletion of *SMN* exon 7 leads to the generation of mice affected with a spinal muscular atrophy phenotype**

To induce neuron-restricted exon 7 deletion, we generated transgenic mice harbouring the Cre recombinase gene driven by the promoter of the rat neuron specific enolase gene. This promoter directs expression specifically to differentiated post-mitotic neurons and neuroendocrine cells (Forss-Petter et al., 1990). Based on the detection of the Cre recombinase transgene, two transgenic lines were selected (NSE-23 and 39, data not shown). To test the efficiency of Cre recombinase activity, transgenic NSE-Cre lines were crossed to the strain harbouring the $SMN^{F7}$ allele and double transgenic mice were selected. Cre-mediated deletion of *SMN* exon 7 was quantified through semi-quantitative PCR amplification analyses of genomic DNA extracted from various tissues. Of the two lines, only one, NSE39-Cre, generated a high level of Cre-mediated exon excision in neuronal tissues and this line was further studied. The efficiency of Cre-mediated deletion was deduced from the ratio of $SMN^{F7}$ to wild type band intensity in various tissues of the double transgenic mice. Cre-mediated deletion of *SMN* exon 7 was high in brain (74%), cerebellum (73%) and spinal cord (56%) while in the other organs including heart, liver and kidney low percentages were observed (figure 3). Ectopic expression of Cre recombinase in non-neuronal tissues suggests that this promoter is subject to positional effects. *In situ* hybridization using a Cre recombinase riboprobe was performed and revealed an expression pattern that was restricted to neurons in the adult spinal cord while in skeletal muscle, no Cre recombinase expression was detected (figure 3). These findings led us to use the reported transgenic line for the conditional targeting of *SMN* in neurons but not in the skeletal muscle.

Transgenic NSE39-Cre mice were crossed with those carrying a $SMN^{F7}$ allele. F1 offspring carrying both the *NSE39-Cre* transgene and ($SMN^{\Delta 7}$) allele were selected and crossed with ($SMN^{F7/F7}$) mice. Twenty eight of 105 (26.6%) mice carried both the *NSE39-Cre* recombinase transgene and the $SMN^{F7/\Delta 7}$ genotype as expected (termed $SMN^{F7/\Delta 7}$, Cre+). Mice carrying this genotype can be easily distinguished from their control littermates by their characteristically impaired movement and tremors both of which become evident from two weeks of age. Furthermore, mice clench their hindlimbs when picked by the tail and hypotonia when suspended on a horizontal thread is apparent and leads to frequent falls (figure 4). In addition, these mice show severe defects in the ability to right themselves when intentionally inverted (figure 4). These mice do not exhibit gait ataxia. Progressively, mice display reduced cage activity and frequently fall when walking. Finally, in addition to these severe motor impairments, mutant animals are smaller and show a 26% reduction in body weight (mean value 6 gr, n= 10 at 12 days of age) compared with mice carrying either $SMN^{F7/\Delta 7}$ genotype but lacking *NSE-Cre* transgene or wild type mice (mean value 8 gr, n= 35 at the same age). Mutant mice exhibit an extremely reduced life expectancy dying at a mean age of 25 days (17 to 36, n=10).

Morphological analysis on transverse sections of several skeletal muscles including gastrocnemius, intercostal and sternomastoid revealed the presence of groups of atrophic muscle fibers in ($SMN^{F7/\Delta 7}$, Cre+) mice when compared with the diameter of fibers in control muscle using hematoxylin and eosin staining (figure 5). The severity of lesions varied from focal areas of severe atrophic muscle fibers to angular fibers intermixed with normal-sized fibers. These findings are consistent with a denervation of skeletal muscles of neurogenic origin. Neither necrosis, inflammatory cell infiltration nor fibrosis was observed (figure 5). To confirm the presence of a muscle denervation process, rhodamine-conjugated $\alpha$-bungarotoxin was used to label the acetylcholine receptors (AChR) at the neuromuscular junction on transverse sections of skeletal muscle. In 2 week-old control mice, AChRs are concentrated at the neuromuscular junction, while a marked extrajunctional labelling was observed in ($SMN^{F7/\Delta 7}$, Cre+) animals thus providing further evidence for a skeletal muscle denervation of neurogenic origin (figure 5).

**Prominent and frequent indentations of the nuclear membrane are associated with the lack of gems and a defect in coiled body formation in motor neuron nuclei**

A morphological analysis on transverse semithin sections of spinal cord was performed on 2 week-old control and ($SMN^{F7/\Delta 7}$, Cre+) mice using toluidine blue staining. In control mice, motor neurons appear as large cells within the anterior horns and show abundant cytoplasm and a prominent nucleolus in a large rounded nucleus (figure 6). In mutant mice, the most striking feature was the pronounced morphological changes of nuclei of these motor neurons (figure 6). A marked increase in toluidine blue staining was observed within the nuclei at the nucleo-cytoplasmic boundary suggesting the presence of indentations of the nuclear membrane (figure 6). These indentations of the nuclear membrane are variable in extent and frequency and are specific to the motor neurons of the mutant mice. In addition, some motor neurons display a granular aspect of the cytoplasm (figure 6). These abnormal nuclei are observed at different levels of the spinal cord including at the cervical, thoracic and lumbar levels. Neither intranuclear nor cytoplasmic neuronal inclusions were apparent from the toluidine blue staining. The nuclei of the neighbouring glial cells did not reveal any abnormal changes. To determine whether these changes were associated with a loss of motor neurons, a quantification of motor neuron number was performed at the lumbar level of the spinal cord using double labelling of nuclei and cytoplasm by DAPI and anti-choline acetyl transferase antibody, an enzyme specific to cholinergic neurons, respectively. At two weeks of age, no significant loss of motor neurons of the anterior horns was detected in mutant mice (data not shown).

To test whether the indentations of the nuclear membrane of motor neurons are associated with an abnormal expression of *SMN* transcript, RT-PCR amplification analysis of RNA extracted from the spinal cord of mutant or wild type mice was performed using primers flanking *SMN* exon 7 (figure 2). A slight increase in transcripts lacking exon 7 was observed in *(SMN$^{F7/\Delta 7}$,* Cre-) mice when compared to the RNA pattern of *(SMN$^{\Delta 7/+}$)* mice suggesting that the *SMN$^{\Delta 7}$* transcript or protein is likely to have an effect on the splicing of transcripts derived from the *SMN$^{F7}$* but not from the wild type allele (figure 2). More importantly, the transcript analysis of *SMN* revealed a marked reduction of the full-length transcript associated with an increased amount of the *SMN$^{\Delta 7}$* transcript in the spinal cord of *(SMN$^{F7/\Delta 7}$ Cre+)* mice when compared with those carrying the other genotypes (figure 2). These results provide evidence of a marked Cre-mediated activity resulting in deletion of exon 7 derived from the *SMN$^{F7}$* allele and leading to a decrease of full length transcript in the spinal cord of *(SMN$^{F7/\Delta 7}$,* Cre+) mice. The absence of Cre recombinase expression in skeletal muscle of *(SMN$^{F7/\Delta 7}$, Cre+)* mice is consistently associated, as expected, with a RNA pattern similar to the one observed in mice lacking the *Cre* transgene (figure 2).

Immunoblotting of proteins prepared from the spinal cord of *(SMN$^{F7/\Delta 7}$, Cre+)* and control mice was performed using the monoclonal antibody directed against the N-terminal part of the SMN protein, 2B1, (Liu and Dreyfuss, 1996). In *(SMN$^{F7/\Delta 7}$, Cre+)* mice, the SMN protein is present with an apparent size and amount similar to that observed in control mice and thus indicating that the mutant *SMN* transcript is translated into a protein (figure 7). To determine whether the sub-cellular localization of the truncated protein is similar to that of the wild type, the monoclonal antibody 2B1 was further used in an immunofluorescence microscopy analysis of spinal cord sections derived from mutant and control mice. In control spinal cord, the 2B1 antibody stained both the cytoplasm and several SMN-containing-gems in the nucleus of motor neurons but not in glial cells (figure 7). In the spinal cord of *(SMN$^{F7/\Delta 7}$, Cre+)* mice, the 2B1 monoclonal antibody stained the cytoplasm but failed to recognize the localization of SMN in gems of motor neurons under the same experimental conditions. These data therefore provide strong evidence for the absence of SMN- containing gems in the motor neuron nuclei of mutant mice (figure 7). To determine whether the absence of SMN in motor neuron nuclei of *(SMN$^{F7/\Delta 7}$,* Cre+) mice is associated with the lack of gems, SIP1, a protein interacting with the N-terminal part of SMN and colocalized with SMN in gems, was immunostained using a monoclonal antibody specific to SIP-1 (2E17). In the spinal cord of control mice, SIP1 antibody stained the cytoplasm and reveals uniform staining of the nucleus in addition to gems in motor neurons (figure 7). In the spinal cord of *(SMN$^{F7/\Delta 7}$, Cre+)* mice, SIP1 antibody labeled both the cytoplasm and the nucleus but it failed to detect gems (figure 7). These findings provide strong evidence of an absence of gems in the motor neurons of mutant mice.

Gems, the structures in which SMN and SIP1 are concentrated in the nucleus, are most often found to be associated with coiled bodies. We therefore tested whether the lack of gems has any effect on the structure or organization of coiled bodies. A polyclonal anti-p80 coilin, a coiled body specific protein (Andrade et al., 1991) was used in an immunofluorescence microscopy analysis. The anti-p80 coilin antibody detects several small foci corresponding to coiled bodies in motor neuron nuclei of control mouse (figure 7). In the spinal cord of *(SMN$^{F7/\Delta 7}$, Cre+)* mice, anti-p80 coilin antibody stained both the cytoplasm and the nucleus. In the nucleus, the antibody labeled several large foci most often merged providing evidence for a defect in the coilin assembly into coiled bodies (figure 7).

**Discussion**

Conditional targeting of mouse *SMN* exon 7 was chosen for two reasons. Firstly, *SMN* exon 7 was targeted since homozygous deletion of this exon is the most frequent mutation found in SMA patients (95%, Lefebvre et al., 1995). Furthermore, small intragenic deletions involving consensus splice sites of introns flanking *SMN* exon 7 were found in some non-deleted patients leading to exon 7 skipping and suggesting a critical function of the corresponding C-terminal end of the protein (Lefebvre et al., 1995; Wirth et al., 1999). In addition, the only difference found at the RNA level between *SMN* and its highly homologous copy, *SMNc,* is the presence of an alternative splicing of exon 7 specific to *SMNc* which leads to a putative truncated protein with a different C terminal end (Lefebvre et al., 1995; Burglen et al., 1996). Secondly, a conditional targeting approach was adopted since the mouse *SMN* gene is not duplicated while the human *SMNc* gene remains functional in SMA patients (Viollet et al., 1997; DiDonato et al., 1997; Schrank et al., 1997). Moreover, strong correlation between the level of protein encoded by *SMNc* gene and the clinical expression of the disease has been demonstrated in SMA providing strong evidence of an important role of the *SMNc* gene (Lefebvre et al., 1997, Coovert et al., 1997). This hypothesis has been reinforced by the early lethality of mouse embryos resulting from conventional knock out of the *SMN* gene (Schrank et al., 1997). These data led us to adopt a conditional mutagenesis approach using the Cre-loxP recombination system.

Initially, mice carrying a loxP-flanked *SMN* exon 7 (*SMN$^{F7}$*) allele were crossed with transgenic mice expressing Cre recombinase under the control of the CMV promoter. Expression of Cre recombinase in the germline allowed the generation of offspring carrying a heterozygous deletion of *SMN* exon 7 in all cell types (*SMN$^{\Delta 7/+}$*). No abnormality

in ($SMN^{\Delta 7/+}$) mice was detected up to 12 months of age. This finding excludes the hypothesis of a dominant negative effect of *SMN* lacking the corresponding C terminal end (SMNΔC15) and is consistent with the recessive mode of inheritance of human SMA. The generation of ($SM^{\Delta 7/+}$) mice allowed us to explore the effect of a homozygous deletion of *SMN* exon 7 in all cell types. Intercrossing ($SM^{\Delta 7/+}$) mice leads to embryonic lethality in the homozygous mutants which is consistent with previous report (Schrank et al., 1997). The complete absence of liveborns homozygous for *SMN* exon 7 clearly shows that this region of the gene plays an essential role in early development. To avoid embryonic lethality, a conditional targeting approach was carried out by using the mice carrying the ($SMN^{F7}$) allele. To restrict *SMN* exon 7 deletion to neurons, the putative target cells in SMA, we generated a transgenic line expressing Cre recombinase under the control of the promoter of the neuron specific enolase gene. This gene has been shown to be expressed from embryonic day 18 in post-mitotic neurons that had completed migration (Forss-Petter et al., 1990). Cre-mediated deletion was detected in several neuronal tissues, including in the spinal cord in which Cre recombinase expression is restricted to neurons. Despite some ectopic expression of Cre recombinase in non-neuronal tissues, the reported transgenic line (NSE39-Cre) has been used for the conditional targeting of *SMN*. $SMN^{F7/F7}$ mice were crossed with the NSE39-Cre transgenic line carrying a heterozygous deletion of *SMN* in order to produce mice carrying a constitutive deletion of exon 7 on one allele, deletion of the other allele ($SMN^{F7}$) depending on Cre recombinase activity. Mice carrying the ($SMN^{F7/\Delta 7}$, *Cre*+) genotype occurred at a rate of 26.6%, close to the expected 25%, and indicate that through the conditional targeting approach we were able to circumvent embryonic lethality. From two weeks of age, the mice carrying ($SMN^{F7/\Delta 7}$, *Cre*+) genotype develop abnormal motor behavior to an extent that allows mutant mice to be easily distinguished from control littermates. Severe defects in both spontaneous and induced motor activities were observed. These defects appear to be progressive as mutant mice develop more pronounced motor disability with age and die usually at a mean age of 25 days. The presence of these motor defects prompted to focus more closely on the neuromuscular system of the mutant mice. Morphological analysis of several skeletal muscles, including those involved in motor activity (gastrocnemius) or respiratory function (intercostal or sternomastoid muscles), revealed the presence of groups of atrophic fibers or angular fibers intermixed with normal-sized fibers. These findings are consistent with muscle denervation of neurogenic origin and are further supported by the presence of a marked extrajunctional expression of AChR, an expression pattern characteristic of a denervation process (Ko et al., 1977). Moreover, the absence of Cre mediated deletion of *SMN* exon 7 in skeletal muscle indicates that the morphological changes in skeletal muscle are secondary to neurogenic lesions.

To determine whether the skeletal muscle denervation observed in the mutant mice is related to motor neuron dysfunction, a morphological analysis of the spinal cords of ($SMN^{F7/\Delta 7}$, *Cre*+) mice was performed. A striking feature was the presence of indentations in the motor neuron nuclear membranes. These abnormal nuclear membrane changes have also been reported to occur with a less pronounced extent in neurodegenerative diseases caused by abnormal polyglutamine-repeat expansion (Davies et al., 1997), however unlike these diseases no neuronal inclusion was observed in *SMN* mutant mice using toluidine blue staining. Electron microscopic examination of motor neurons should contribute to further investigate the morphological changes of the nuclear membrane. It is clear that the presence of indentations in the nuclear membranes of motor neurons is associated with pronounced neuronal dysfunction as demonstrated by the denervation of skeletal muscle fibers, the targeted cells of motor neurons, despite the absence of motor neuron loss. These results strongly suggest that the neuronal nucleus is the primary site involved in neuronal dysfunction in the mutant mice. Previous reports have suggested that a defect of muscular origin could be responsible for motor neuron degeneration in SMA (Henderson et al. 1987, Braun et al., 1995). Our results demonstrate that a *SMN* gene defect directed to neurons but not to skeletal muscle leads to a SMA phenotype *in vivo* indicating that motor neurons are the primary target of the *SMN* gene defect in SMA. Although unlikely, it is possible that the locomotor impairment of the mutant mice is not only the consequence of motor neuron dysfunction in the spinal cord but is also due to the involvement of other neuronal cell types. Morphological examination of other neuronal cell populations should contribute to answering this question. However, the mutant mice exhibit consistent and clear evidence of severe skeletal muscle denervation processes which lead to muscle paralysis associated with motor neuron dysfunction, a constant feature found in human SMA (Byers and Banker, 1961).

The pronounced effect of SMNΔC15 on the organization of both gems and coiled bodies was unexpected. The dramatic reduction of SMNΔC15 within the motor neuron nuclei of mutant mice indicates that the C terminal end of SMN (amino acids 274 to 288) represents an important domain required for the SMN targeting into gems. Interestingly, the absence of SIP1 assembly in gems strongly suggests that SMN is involved in the normal pathway of gem formation *in vivo*. Previous studies indicated that gems were distinct from coiled bodies though they were most often found to be closely associated (Liu and Dreyfuss, 1996). The lack of gems in mutant mice led us to examine coiled body organization. The presence of large nuclear foci of coilin most often merged associated with a cytoplasmic staining of coilin indicates that the lack of SMN and/or gems within the nucleus has a pronounced effect on coiled body formation. A likely possibility is that wild type SMN is involved in the biogenesis of gem, the gem formation being a prerequisite for coilin targeting into coiled bodies. Our data could also suggest that coiled bodies and gems are the same structure, SMN being involved in the normal pathway of coiled body/gem formation. Therefore, the generation of a mouse model carrying the most frequent mutation found in human SMA allowed to determine that SMNleads to a defect in nuclear targeting of SMN which cannot proceed further the normal pathway of gem/coiled body formation. The present study contributes thus to dissect the pathogenic mechanism resulting in SMA. Although the lack of the SMN protein in nuclei has been previously reported so far in SMA patients (Lefebvre et al., 1997), the associated morphological nuclear changes occuring in response to the lack of SMN have not been reported in human SMA. This suggests that these morphological changes are likely to represent the early events of neuronal degeneration which lead later to motor neuron loss, a late manifestation such as that found in autopsy material of SMA patients (Byers and Banker, 1961).

Recently, aberrant RNA processing was reported in amyotrophic lateral sclerosis, a neurodegenerative disease characterized by selective degeneration of upper and lower motor neurons (Lin et al., 1998). Multiple abnormal mRNAs of the excitatory amino acid transporter 2, an astroglial glutamate transporter, were identified with no mutation in the corresponding gene. These data were suggestive of the presence of a defect in an unknown regulatory factor required for the normal splicing of this transcript. The present study shows a close correlation between the defect of a component of the spliceosomal complex and motor neuron dysfunction characteristic of spinal muscular atrophy. This link provides further evidence that defects in RNA processing represent an important mechanism in the development of neurodegenerative diseases. Therefore, the mutant mice reported in this study represent an appropriate animal model for understanding the role of SMN in normal motor neuron function as well as in pathophysiology. Finally, this animal model is likely to become an important tool both for the design of therapeutic strategies to counter human SMA and to aid in increasing the understanding of the events leading to neuronal degeneration.

II <u>GENERATION OF MICE AFFECTED WITH SEVERE MUSCULAR DYSTROPHIC PHENOTYPE</u>

**Homozygous deletion of SMN exon 7 restricted to skeletal muscle leads to a severe muscular dystrophic phenotype.**

The transgenic HC79-Cre mouse line (Miniou P, Tiziano D, Frugier T, Roblot N, Le Meur M and Melki J. Gene targeting restricted to mouse striated muscle lineage. Nucl. Acids Res. 1999, 27:27-31) was crossed to that carrying a $SM_N\Delta7$ allele. F1 offspring carrying both the HC79-Cre transgene and ($SM_N\Delta7$) allele were selected and crossed with ($SMN^{F7/F7}$) mice. Twenty five percent of mice carried both the *HC79-Cre* recombinase transgene and the $SMN^{F7/\Delta7}$ genotype as expected (termed $SMN^{F7/\Delta7}$ *Cre*+) Mice did not show any abnormal phenotype within the three first weeks of age. Defects in both spontaneous and induced motor activities start from three weeks of age to an extent that allows mutant mice of both sexes to be easily distinguished from control littermates. Progressively, mice display reduced cage activity and frequently fall when walking and die at a mean age of 31 days (28-37 days, n=10). The presence of these severe motor defects prompted us to focus more closely on the neuromuscular system of the mutant mice. Morphological analysis of several skeletal muscles including gastrocnemius, biceps brachii and triceps suralis revealed the presence of markedly hypertrophic fibers, excessive variation in fibber size and internal nuclei suggesting a muscular dystrophic process. In addition, degenerative changes with necrosis associated with inflammatory cell infiltration in interstitial tissue can be observed. No fibrosis was observed. These changes are characteristic features of muscular dystrophy.

To test whether the muscular dystrophic process is associated with an abnormal expression of *SMN* transcript, RT-PCR amplification analysis of RNA extracted from the skeletal muscle of mutant or wild type mice was performed using primers flanking *SMN* exon 7. The transcript analysis of *SMN* revealed a dramatic reduction of the full-length transcript associated with an increased amount of the $SM_N\Delta7$ transcript in the skeletal muscle of ($SMN^{F7/\Delta7}$ HC79-*Cre*+) mice when compared with those carrying the other genotypes. These results provide evidence of a marked Cre-mediated activity resulting in deletion of exon 7 derived from the $SMN^{F7}$ allele and leading to a dramatic decrease of full length transcript in the skeletal muscle of ($SMN^{F7/\Delta7}$ HC79-*Cre*+) mice.

Previous studies of HC79-Cre transgenic mouse line revealed a Cre recombinase expression starting at embryonic day 9.5 suggesting that Cre-mediated deletion should occur during embryogenesis. Surprisingly, the onset of symptoms of ($SMN^{F7/\Delta7}$ HC79-*Cre*+) mice occur around 3 weeks of age. These data suggest that SMN may have a critical role in development or differentiation of skeletal muscle during post-natal period.

The observation of a dystrophic muscle pattern led us to study the dystrophin, the product of the gene mutated in the most frequent muscular dystrophy in human, Duchenne or Becker muscular dystrophies. Monoclonal antibodies against the C-terminus of the dystrophin revealed a marked decrease or the complete lack of dystrophin at the muscle membrane. Interestingly, in some muscle fibers, antibody detected a dystrophin labelling in the cytoplasm. Finally, very rare muscle fibers display a normal pattern of expression of dystrophin which may be related to the absence of Cre recombinase activity in some muscle fibers as previously reported in HC79-Cre mouse line (Miniou P; Tiziano D, Frugier T, Roblot N, Le Meur M and Melki J. Gene targeting restricted to mouse striated muscle lineage. Nucl. Acids Res. 1999, 27:27-31). Monoclonal antibodies directed to the N-terminus of dystrophin revealed the same abnormal pattern of expression. These date suggest that a primary or secondary defect of dystrophin is associated with the muscular

dystrophic phenotype of (SMN$^{F7/\Delta7}$, HC79-Cre+) mice.

In order to test whether the SMN gene defect leads to splicing defect of RNA as previously suggested *in vitro,* analysis of dystrophin transcripts will be carried out by both semi-quantitative RT-PCR and northern blot analyses. The dystrophin gene is one of the most large known genes and several intronic sequences are larger than 50 Kb. In addition, alternative splicing of dystrophin transcripts have been already identified and characterized. Under the hypothesis that SMN is involved in RNA maturation *in vivo,* large intronic sequences may require a large amount of SMN for accurate splicing reaction. Therefore, genes with large intronic sequences such as dystrophin gene may represent good targets for SMN gene defect. To test this hypothesis, RT-PCR analysis using primers chosen in exons and flanking large intronic sequences will be studied in affected (SMN$^{F7/\Delta7}$, HC79-Cre+) and control mice. A primary defect of dystrophin RNA in mutant mice will be investigated by a comparative analysis of dystrophin RNA with transcripts derived from other genes transcribed in skeletal muscle, or an analysis of dystrophin in skeletal muscle compared with dystrophin expression in the other tissues.

The presence of a marked defect of the dystrophin protein associated with a muscular dystrophy phenotype in (SMN$^{F7/\Delta7}$, HC79-Cre+) mice is unexpected. These date suggest that defect of the SMN gene may modulate the clinical severity of the human spinal muscular atrophy phenotype by a direct skeletal muscular involvement. Interestingly, about one fourth of the patients affected with the mildest form of childhood SMA (Type III or Kugelberg Welander disease) exhibit a hypertrophy of the calves, a feature similar to that observed in Duchenne or Becker muscular dystrophies (Bouwsma G, Vanwijngaarden: Spinal muscular atrophy and hypertrophy of the calves. J. *Neurol.* Sci. 44: 275, 1980). In type III SMA but not in infantile SMA (type I or II), one can observe markedly hypertrophic fibers, excessive variation in fiber size and internal nuclei. Degenerative changes with necrosis, regenerative fibers associated with proliferative interstitial connective tissue can be observed (Mastaglia FL, Walton JN: Histological and histochemical changes from cases of chronic juvenile and early adult spinal muscular atrophy (the Kugelberg-Welander syndrome. J. *Neurol. Sci.* 12: 15, 1971). Since these changes are characteristic features of primary myopathies, they have been interpreted as "pseudomyopathic" changes. In addition, these myopathic changes are usually found in patients with high serum levels of creatinphosphokinase (CK) activity and may suggest the presence of a myopathic process. Studying dystrophin at the transcriptional and protein level in human SMA should provide novel insight into the pathogenesis of human SMA.

Finally, the (SMN$^{F7/\Delta7}$, HC79-Cre+) mice may represent a valuable animal model of muscular dystrophy.

III USES OF THE GENERATED MICE MODELS AND CELL CULTURES DERIVED THEREFROM

**A. Use of mouse model called NSE39-CreSMN F7/∆7**

A1. The generation of an animal model of spinal muscular atrophy should contribute to a better knowledge of the molecular mechanism resulting in degeneration of motor neurons or other neuronal populations by identifying i) transcripts up or down regulated in affected or non affected spinal cord tissues ii) abnormal processed transcripts i.e. abnormal alternative splicing products or splicing defect of small or large intronic sequences of transcripts. Transcript analysis will be performed from RNA extracted from affected spinal cord tissues of (NSE39-Cre SMN F7/∆7) mice (as obtained according to the description in the above Section I) and compared to RNA extracted from control spinal cord.

Mice carrying the genotype SMNF7/SMN∆7 and expressing the Cre recombinase transgene under the control of the neuron specific enolase promoter (NSE39-Cre) will be selected. The selection is based on genotype analysis at the SMN locus and on the detection of the Cre recombinase transgene. Mice carrying the wild type SMN locus and lacking the Cre recombinase transgene will be used as controls. Total RNA will be extracted from non-affected or affected spinal cord samples using Trizol procedure (GIBCO/BRL) or other methods.

Differential display analysis can be used to compare the RNA expression pattern from non affected or affected spinal cord samples using the method described by P. Liang and Pardee, 1992 (Differential display of eukaryotic messenger RNA by means of the polymerase chain reaction, 257: 967-971) and refined by Liang et a. 1993 (Distribution and cloning of eukaryotic mRNAs by means of differential display: refinement and optimization, Nuci. Acids Research, 21:3269-3275). Differently expressed bands derived from either affected and non affected spinal cord samples will be selected, cut, reamplified with the same primers before cloning into pBS plasmid vector or other vectors and sequencing. Northern blot or semi-quantitative RT-PCR analyses of RNA extracted from affected and non affected spinal cord tissues will enable either to confirm the differential expression of RNA or to detect RNA molecules abnormal in size using the selected cDNA products as probe. Sequencing analysis will be performed on double strand plasmid DNA with oligonucleotides derived from the vector and analyzed on DNA automated sequencer. In order to identify homologous or identical nucleotide or amino acid sequences, data will be compared with those contained in the nucleotide and protein databases. The same procedure will be applied to identify abnormal processed RNA molecules by detecting non spliced products or skipping of exons.

High density filters carrying cDNA molecules obtained by PCR amplification or plasmid DNA preparation from mouse brain or spinal cord cDNA libraries can be used for identifying differentially expressed RNA. First strand synthesis of RNA extracted from affected and non affected spinal cord tissues will be PCR amplified and labeled using randomly chosen primers and the PCR products of affected and non affected spinal cord tissues will be hybridized to filters. Comparative analysis of signals will enable to select candidate cDNA molecules. Sequencing analysis will be performed on double strand plasmid DNA with oligonucleotides derived from the vector and analysed on DNA automated sequencer. In order to identify homologous or identical nucleotide or amino acid sequences, data will be compared with those contained in the nucleotide and protein data bases. The same procedure will be applied to identify abnormal processed RNA molecules by detecting non spliced products or skipping of exons.

Serial analysis of gene expression (SAGE) is another method which should be able to identify differently expressed RNA molecules. This method has been reported by Velculescu et al. 1995 (Serial analysis of gene expression. Science 270, 487-7) and reviewed by Bertelsen and Velculescu, 1998 (High throughput gene expression analysis using SAGE. Drug Discovery Today 3: 152-159).

These investigations should contribute to identify i) molecules targeted by the SMN gene defect ii) the molecules involved or associated with the neurodegenerative process. The identification and characterization of these molecules will be carried out using genetic, transcriptional and biochemical analyses. Genetic characterization will be achieved by data base searches or genomic library screening. RNA expression will be studied by northern blot, *in situ* hybridization or RT-PCR experiments using the selected cDNA molecules as probe or primers specific for the identified molecules. Characterization of the protein products will be carried either by generating polyclonal or monoclonal antibodies against the protein or part of the protein products (i.e. synthetic peptide). These antibodies will allow immunolocalization of the protecin on tissue sections or cell culture, immunoblot analysis and immunoprecipitation experiments.

Defective molecules resulting from the SMN gene defect can represent new targets for therapeutical strategy using gene therapy or pharmacological approaches. Abnormal spliced products, the lack of the marked reduction of transcripts leading to a protein defect can be compensated by administration of cDNA or purified protein.

Therefore, identifying these molecules can help in finding therapy in SMA as well as diseases closely related to SMA such as arthrogryposis or amyotrophic lateral sclerosis or diseases characterized by a nerodegenerative process i.e. Parkinson disease, Alzheimer disease etc.

A2. Comparative protein analysis between affected and non affected spinal cord tissues can be performed using 2-D gel electrophoresis (Hanash SM, Strahler JR, Neel JV, Hailat N, Melhem R, Keim D, Zhu XX, Wagner D, Gage DA, Watson JT. Highly resolving two-dimensional gels for protein sequencing. Proc Nati Acad Sci U S A 1991; 88-5709-13). Microsequencing or mass spectrometric analysis of differently expressed proteins will enable to identify part of the amino acid sequence and therefore the corresponding nucleotide sequence. Further investigations at the genetic level, RNA and protein levels will be achieved as described above.

A3. Deletion of SMN exon 7 is restricted to neurons. Skeletal muscle, glial cells or ependymal cells are not affected. Interestingly, skeletal muscle denervation is markedly pronounced despite the absence of loss of motor neurons.

The presence of motor neurons in mutant mice suggest that either the motor neuron loss is a late manifestation of the disease or there is a regeneration of motor neurons form unaffected cells i.e. glial cells or ependymal cells. These potential progenitors are not affected since the Cre recombinase is not expressed as determined by in situ hybridization experiments using Cre recombinase antisense as probe (see experimental data below). Therefore, these mutant mice represent a valuable model to test the capacity of progenitor cells to differentiate into motor neurons.

To test this capacity, analyses of transcripts or proteins involved in or associated with motor neuron division or differentiation will be performed using biochemical, immunohistochemical and in situ hybridization experiments on spinal cord of mutant mice. Islet-1, Islet-2, Lim-1/2, Lim-3, Cek-8, Mek-4, BMP2, Twh which are proteins involved in such process will be investigated in mutant mice (DeLapeyriere and Henderson, Current Opinion in Genetics and Development, 1997, 7:642-650 and Morrisson SJ et al. Prospective identification, isolation by flow cytometry and in vivo self-renewal of multipotent mammalian neural crest stem cells, Cell, 1999, 96: 737-749).

## B. Derivatives of the mutant mice

The mutant mice harbouring the genotype SMN F7/A7 or SMN F7/F7 and carrying or lacking the NSE 39-Cre recombinase transgene will allow primary cultures of neurons of the spinal cord using the method as previously described by Camu W, Henderson CE (Purification of embryonic rat motoneurons by panning on a monoclonal antibody to the low-affinity NGF receptor. J Neurosci Methods 1992 44:59-70). Transformation of neurons or any other cells will be undertaken for instance by using the SV40 T antigen gene that is able to immortalize cells.

The same procedure may be applied to obtain cultures of cells derived from mutant mice harbouring the genotype SMN F7/Δ7 or SMN F7/F7 and carrying or lacking the HC79-Cre recombinase transgene, especially to produce cell cultures of muscle tissues, particularly skeletal muscle tissue.

The cells can be motor neurons or other neuronal cell populations or muscular cells derived from the mutant mouse. Primary cultures or transformed derivatives will be used for identifying transcripts or proteins up or down regulated by the SMN gene defect. Strategies similar to those described above in relation with mutant mice can be carried out.

**C. Therapeutic approaches in animal models.**

SMA (NSE 39-Cre SMN F7/Δ7):

This mouse model or cells derived therefrom, especially those of neuronal tissue, represents an appropriate tool for therapeutic trials. The efficacy of therapeutic trials will open the possibility to undertake therapeutic trials in human type I, II, III SMA or the adult form - and related neurodegenerative diseases such as amyotrophic lateral sclerosis SLA or arthrogryposis.

(NSE 39-Cre SMN F7/Δ7)

This mouse model or cells derived therefrom, especially those of neuronal tissue represents an appropriate tool for therapeutic trials. The efficacy of therapeutic trials will open the possibility to undertake design therapeutic strategies and therapeutic trials in muscular diseases.

**C1 - Methods**

■ Oral, intraveinous, intra-muscular, intra-peritoneal injection or injection to cerebral spinal fluid can be the routes for drug administration.
■ Viral or synthetic vectors, as well as encapsulated or non encapsulated cells from the mouse of from another organism can be used to deliver the drugs (Bordet T et al., Adenoviral cardiotrophin-1 gene transfer protects pmn mice from progressive motor neuronopathy. J Clin Invest 1999 104:1077-85. Haase G. et al. Therapeutic benefit of ciliary neurotrophic factor in progressive motor neuronopathy depends on the route delivery. Ann Neuro 1999 45:296-304. Haase G et al. J Neurol Sci 1998 160 Suppl 1:S97-105 Adenovirus-mediated transfer of the neurotrophin-3 gene into skeletal muscle of pmn mice: therapeutic effects and mechanisms of action. Haase G et al. Gene therapy of murine motor neuron disease using adenoviral vectors for neurotrophic factors. Nat Med 1997 3: 429-36, Sagot Y et al. Polymer encapsulated cell lines genetically engineered to release ciliary neurotrophic factor can slow down progressive motor neuronopathy in the mouse. Eur J Neurosci 1995 7:1313-22).
■ Unique or repeated administration(s) can be necessary.
■ The drugs will be delivered before the onset of symptoms or after the onset of symptoms of both.
■ The efficacy will be checked by using morphological, motor behaviour analyses as well as weight and age of survival of mutant mice.

Morphological analysis : Spinal cord (L4 lumbar segment) and skeletal muscle (gastrocnemius) will be embedded in OCT compound (Miles Laboratory, Incorporated) and frozen at -80°C in isopentane cooled on liquid nitrogen. Cryostat sections of 10 μm will be mounted on gelatinized coverslips and air-dried. In order to judge the number of motor neurons in spinal cord. Nissl staining will be performed using 0.1% cresyl violet for 10 min. Nissl staining will allow to identify motor neurons that display very prominent cytoplasmic staining and a strongly labeled nucleolus. Muscle tissue will be analyzed by Haematoxylin-Eosin staining which allows to evaluate the morphological aspect of the muscle and to define the size of the fibers. Motor neuron loss and muscle denervation processes will then be quantified using imaging densitometer apparatus (Biorad). Sprouting from axons of surviving motor neurons will be checked by labelling both axons (using for example neurofilament antibodies) and neuromuscular junctions (using for example rhodamine-conjugated α-bungarotoxin).

Behavioural analysis Spontaneous locomotor activity of mice will be assessed by an infrared beam activity monitor. One mouse each is placed in a standard microisolator cage with enough food and water. Infrared beams will be broken by mice normally walking the length of the cage. The apparatus simultaneously and continuously monitored beam breaks for 16 cages. Day and night are defined by a periodic 12 hr light and dark cycle, respectively. Data are simultaneously recorded by an IBM personal computer programmed to store data at 2 min intervals for each cage.

These date are converted to an Asci text file and analysed. Induced motor activity of mice will be assessed by rota-rod test. The rota-rod test will be performed by placing each mouse on a rotating drum and measuring the time the animal is able to maintain its balance on the rode. Mice will be placed on a 4 cm diameter rotarod rotating at 2.5 rpm, 30 cm above a padded cage bottom. Mice will be scored as either falling or not falling within a 60 sec trial.

**C2 - Molecules selected for therapy**

Such molecules that would be candidates for therapeutic strategies may encompass gene or protein products.

For example, they may be selected in groups including :

∗ Neurotrophic factors (NT3, cardiotrophin or other cytokines, GDNF or other molecules with such biological properties) ;
∗ SMN from human or from other species. SMN can be used alone or fused to the TAT motif of HIV in order to induce transduction into cells and nucleus as recently described (Schwarze SR et al., In vivo protein transduction: delivery of a biologically active protein into the mouse. Science 1999 285: 1569-72) or other peptides able to induce transduction of protein or DNA into cells. SMN can be fused to consensus signal sequence in order to induce SMN secretion (Gascuel O et al, Protein export in prokaryotes and eukaryotes: indications of a difference in the mechanism of exportation. J Mol Evpm 1986; 24:130-42);
∗ Molecules involved in apoptosis, in particular inhibitors of the apoptosis;
∗ Molecules identified or derived from evolution of differential RNA or protein expression patterns.

**D. Targeting SMN exon 7 deletion in other tissues**

Mouse lines carrying SMN F7/or SMN F7/SMN F7 or SMN F7/A7 are established and can be used to direct deletion of SMN exon 7 in other tissues i.e. skeletal muscle, specific neuronal or non-neuronal populations. Deletion of SMN exon 7 restricted to other tissues can be achieved using mouse transgenic line expressing the Cre recombinase in the appropriate tissues and crossed to mice carrying the genotype described above.

E. Mouse line carrying the Cre recombinase gene driven by the neuron specific enolase promoter can be used to target activation or inactivation of a given gene or sequence flanked by two loxP sites placed in the same orientation through homologous recombination in embryonic stem cells or microinjection in zygote pronucleus of mice and reimplantation into pseudopregnant foster mothers. In addition, this mouse line can be used to target deletion of an exon or a group of exons of the mouse SMN gene.

F. Mouse model called NSE 39-Cre SMN$^{F7/\Delta7}$ can be useful to know whether the neurodegenerative process found in SMA can be reversed. The observation of a severe denervation process in mouse model without marked loss of motor neurons should allow to prevent neuronal death. Conditional expression of an intact SMN gene at different times of the disease course in the mouse models of SMA should allow to answer this question. Conditional expression of an intact gene can be achieved using the tetracycline-controlled transactivator (tTA) or the reverse (rtTA) system. In this system, tetracycline administered in the food can suppress or induce SMN transgene expression driven by the tetO promoter (Gossen M, Bujard H, Tight control of gene expression in mammalian cells by tetracycline-responsive promoters, Proc Nati Acad Sci USA 1992 89: 5547-51, Kistner A, Gossen M, Zimmermann F, Jerecic J; Ullmer C, Lubbert H, Bujard H, Doxycycline-mediated quantitative and tissue-specific control of gene expression in transgenic mice, Proc Nati Acad Sci USA 1996;93:10933-8).

By temporally manipulating the expression of the SMN transgene with tetracycline, we could obtain evidence that the SMA phenotype observed in (NSE39-Cre+, SMN$^{F7/\Delta7}$) mice can be reserved. Two transgenic mouse lines will be established. One carrying the SMN cDNA from human or other organisms under the control of the tetO promoter, the other expression the tTA or rtTA transactivator under the control of a neuronal promoter such as neuron specific enolase gene promoter (Forss-Petter, S., Danielson, P.E., Catsicas, S., Battenberg, E., Price J., Nerenberg, M. and Sutcliffe, J.G. (1990) Transgenic mice expressing β-galactosidase in mature neurons under neuron-specific enolase promoter control. *Neuron,* 5, 187-197).

The same system can be used to try to reverse the phenotype of HC79-cre, SMNF7/D7.

In the HC79-Cre, SMNF7/D7 mice, another system to reverse the phenotype will consist of the inducible expression of the dystrophin cDNA (and not the gene) through transgenesis or viral or synthetic vectors. This strategy will enable to prevent the disease phenotype if a splicing defect of dystrophin transcripts is confirmed. In addition, this strategy will enable to see whether dystrophin is a target gene of the SMN gene defect.

SMN gene delivery can be also achieved using viral or synthetic vectors as described in section C. Using these strategies, we should be able to determine at which time of the disease progression, the SMA phenotype can be reversed or stabilized. These date should provide important information for therapy of the human disease.

## References

Andrade, L. E., Chan, E. K. , Raska, I., Peebles, C. L., Roos, G., Tan, E. M. (1991) Human autoantibody to a novel protein of the nuclear coiled body: immunological characterization and cDNA cloning of p80-coilin. J. Exp. Med. 173, 1407-19.

Braun, S., Croizat, B., Lagrange, M. C. , Warter, J. M., Poindron, P. (1995). Constitutive muscular abnormalities in culture in spinal muscular atrophy. Lancet 345, 694-5.

Byers, R.K., and Banker, B. Q. (1961). Infantile spinal muscular atrophy. Archives of Neurology 5, 140-164.

Bürglen, L., Lefebvre, S., Clermont, O., Burlet, P., Viollet, L., Cruaud, C., Munnich, A. and Melki, J. (1996). Structure and Organisation of the Human Survival Motor Neurone *(SMN)* gene. Genomics 32, 479-482.

Coovert, D. D., Le, T.T., McAndrew, P. E., Strasswimmer, J., Crawford, T. O., Mendell, J. R., Coulson, S., Androphy, E. J. , Prior, T. W. and Burghes, A. H. M. (1997). The survival motor neuron protein in spinal muscular atrophy. Hum. Mol. Genet. 6, 1205-1214.

Czeizel, A. and Hamula, J. (1989). Selective and non-selective susceptibility for muscle fiber types. J. Med. Genet. 26, 761-763.

Davies, S. W., Turmaine, M., Cozens, B.A., DiFiglia, M., Sharp, A.H., Ross, C.A., Scherzinger, E., Wanker, E. E., Mangiarini, L., Bates, G.P. (1997). Formation of neuronal intranuclear inclusions underlies the neurological dysfunction in mice transgenic for the HD mutation. Cell 90, 537-48.

DiDonato, C. J., Chen, X. N., Noya, D., Korenberg, J. R., Nadeau, J. H., Simard, L. R. (1997). Cloning, characterization, and copy number of the murine survival motor neuron gene: homolog of the spinal muscular atrophy-determining gene. Genome Res. 7, 339-52.

Dupe, V., Davenne, M., Brocard, J., Dolle, P., Mark, M., Dierich, A., Chambon, P., Rijli, F. M. (1997). In vivo functional analysis of the Hoxa-1 3' retinoic acid response element (3'RARE). Development *124,* 399-410.

Fischer, U., Liu, Q., Dreyfuss, G. (1997). The SMN-SIP1 complex has an essential role in spliceosomal snRNP biogenesis. Cell *90*, 1023-9.

Forss-Petter, S., Danielson, P. E., Catsicas, S., Battenberg, E., Price J., Nerenberg, M., Sutcliffe, J. G. (1990). Transgenic mice expressing (3-galactosidase in mature neurons under neuron-specific enolase promoter control. Neuron *5*, 187-97.

Gu, H., Marth, J. D., Orban, P.C., Mossmann, H., and Rajewski, K. (1994). Deletion of a DNA polymerase gene segment in T cells using cell type-specific gene targeting. Science. *265,* 103-106

Henderson, C. E., Hauser, S. L., Huchet, M., Dessi, F., Hentati, F., Taguchi, T., Changeux, J. P., Fardeau, M. (1987). Extracts of muscle biopsies from patients with spinal muscular atrophies inhibit neurite outgrowth from spinal neurons. Neurology 37, 1361-4

Ko, P. K., Anderson, M. J., Cohen, M. W. (1977). Denervated skeletal muscle fibers develop discrete patches of high acetylcholine receptor density. Science *196,* 540-542

Lefebvre, S., Bürglen, L., Reboullet, S., Clermont, O., Burlet, P., Viollet, L., Benichou, B., Cruaud, C., Millasseau, P., Zeviani, M., Le Paslier, D., Frézal, J., Cohen, D., Weissenbach, J., Munnich, A. and Melki, J. (1995). Identification and characterization of a spinal muscular atrophy-determining gene. Cell *80*, 155-165.

Lefebvre, S., Burlet, P., Liu, Q., Bertrandy, S., Clermont, O., Munnich, A., Dreyfuss, G. et Melki, J. (1997). Correlation of severity with the SMN protein level in spinal muscular atrophy. Nature Genetics *16,* 265-269.

Lewin, B. (1995). Genes for SMA: multum in parvo. Cell 80, 1-5.

Li Hsiu Mei Hsieh et al (January 2000), Nature Genetics, vol. 24.

Lin, C. L., Bristol, L. A., Jin, L., Dykes-Hoberg, M., Crawford, T., Clawson, L., Rothstein, J. D. (1998). Aberrant RNA processing in a **neurodegenerative** disease: the cause for absent EAAT2, a glutamate transporter, in amyotrophic lateral sclerosis. Neuron 20, 589-602.

Liu, Q. and Dreyfuss, G. (1996). A novel nuclear structure containing the survival of motor neuron protein. Embo Journal 15, 3555-3565.

Liu, Q., Fischer, U., Wang, F., Dreyfuss, G. (1997). The spinal muscular atrophy disease gene product, SMN, and its associated protein SIP1 are in a complex with spliceosomal snRNP proteins. Cell 90, 1013-21.

Lorson, C. L., Hahnen, E., Androphy, E. J., Wirth, B. (1999). A single nucleotide in the *SMN* gene regulates spcing and is responsible for spinal muscular atrophy. Proc. Natl. Acad. Sci. U.S.A. 96, 6307-11.

Lorson, C. L., Strasswimmer, J., Yao, J. M., Baleja, J. D., Hahnen, E., Wirth, B., Le, T., Burghes, A. H., Androphy, E.J. (1998). SMN oligomerization defect correlates with spinal muscular atrophy severity. Nat. Genet. 19, 63-6.

Melki, J., Lefebvre, S., Burglen, L., Burlet, P., Clermont, O., Millasseau, P., Reboullet, S., Zeviani, M., Le Paslier, D., Cohen, D., Weissenbach, J. and Munnich, A. (1994) *De novo* and inherited deletions in spinal muscular atrophies. Science. *264*, 1474-1477

Melki, J. (1997). Spinal muscular atrophy. Curr. Opin. Neurol. *10,* 381-5.

Munsat, T.L. (1991). Workshop report: International SMA Collaboration. Neuromusc. Disord. 1, 81.

Niederreither, K., ad Dollé, P. (1998). *In situ* hybridization with 35S-labeled probes for retinoid receptors. Methods in Molecular Biology. Edited by C. P. F. Redfern. Humana Press Inc.89, 247-267.

Pearn, J. (1973). The gene frequency of acute Werdnig-Hoffmann disease (SMA type I). A total population survey in North-East England. J. Med. Genet. *10,* 260-265.

Pearn, J. (1978). Incidence, prevalence and gene frequency studies of chronic childhood spinal muscular atrophy. J. Med. Genet. 15, 409-413.

Pellizzoni, L., Kataoka, N., Charroux, B., Dreyfuss, G. (1998). A novel function for SMN, the spinal muscular atrophy disease gene product, in pre-mRNA splicing. Cell 95, 615-24.

Roberts, D. F., Chavez, J. and Court, S. D. M. (1970). The genetic component in child mortality. Arch. Dis. Child. 45, 33-38.

Sauer, B., and Henderson, N. (1988). Site-specific DNA recombination in mammalian cells by the Cre recombinase of bacteriophage P1. Proc. Natl. Acad. Sci. U.S.A. 85, 5166-5170.

Schrank, B., Götz, R., Gunnersen, J. M., Ure, J. M., Toyka, K. V., Smith, A. G., and Sendtner, M. (1997). Inactivation of the survival motor neuron gene, a candidate gene for human spinal muscular atrophy, leads to massive cell death in early mouse embryos. Proc. Natl. Acad. Sci. U.S.A. 94, 9920-9925.

Sternberg, N., and Hamilton, D. (1981). Bacteriophage P1 site- specific recombination. I. Recombination between loxP sites. J. Mol. Biol. *150,* 467-486.

Viollet, L., Bertrandy, S., Bueno-Brunialti, A. L., Lefebvre, S., Burlet, P., Clermont, O., Cruaud, C., Guenet, J. L., Munnich, A., Melki, J. (1997). cDNA isolation, expression, and chromosomal localization of the mouse survival motor neuron gene (*SMN*). Genomics *40,* 185-8.

Wirth, B., Herz, M., Wetter, A., Moskau, S., Hahnen, E., Rudnik-Schoneborn, S., Wienker, T., Zerres, K. (1999). Quantitative analysis of survival motor neuron copies: identification of subtle *SMN1* mutations in patients with spinal muscular atrophy, genotype-phenotype correlation, and implications for genetic counseling. Am. J. Hum. Genet. 64; 1340-56.

**Claims**

1. Non-human mammal carrying a conditional mutation in the *SMN* gene, said mutation enabling a tissue-specific expression pattern of a mutated SMN protein.

2. Non-human mammal according to claim 1, wherein the conditional mutation in the *SMN* gene is a mutation in exon 7 that enables the expression of a SMN protein having a truncated C-terminal end.

3. Non-human mammal according to anyone of claims 1 or 2 which expresses an SMN protein lacking the 15 C-terminal amino-acid residues (SMNΔC15).

4. Non-human mammal according to claim 1 or 2, wherein the conditional mutation in the *SMN* gene is a deletion of exon 7.

5. Non-human mammal according to anyone of claims 1 to 4, which is heterozygous for the *SMN* gene (*SMN*$^{F7/\Delta7}$), carrying one allele having a lox P-flanked exon 7 (F7) and one allele having a deleted exon 7 (A7), said mammal further expressing the Cre recombinase under the control of tissue-specific regulation sequences.

6. Non-human mammal according to anyone of claims 1 to 5, wherein the conditional mutation of the SMN gene affects the endogeneous sequence of the gene.

7. Non-human mammal according to anyone of claims 1 to 6, wherein the mutation of the *SMN* gene is a conditional homozygous mutation, which expression is restricted to a neuronal tissue.

8. Non-human mammal according to anyone of claims 1 to 6, wherein the mutation of the *SMN* gene is a conditional homozygous mutation, which expression is restricted to skeletal muscle tissue.

9. Non-human mammal according to anyone of claims 1 to 8, wherein the conditional mutation is obtained by a recombination system, driven by tissue specific regulation sequences.

10. Non-human mammal according to anyone of claims 1 to 4 or 6 to 9, which is selected from non-human mammal wherein one allele of the *SMN* gene carries a loxP-flanked *SMN* exon 7 (SMN$^{F7}$), said non-human mammal further carrying a sequence encoding the Cre-recombinase under the control of tissue specific regulation sequences, or from non-human mammal wherein one allele of the *SMN* gene carries a deleted *SMN* exon 7 (SMN$^{\Delta 7}$), said non-human mammal further carrying a sequence encoding the Cre-recombinase under the control of tissue specific regulation sequences .

11. Non-human mammal according to anyone of claims 1 to 9, wherein one allele of the *SMN* gene carries a deletion of exon 7.

12. Non-human mammal according to anyone of claims 1 to 11, which exhibits a SMA phenotype.

13. Non-human mammal according to anyone of claims 1 to 11, which exhibits a muscular dystrophic phenotype.

14. Non-human mammal according to anyone of claims 1 to 13 which is a rodent.

15. Non-human mammal according to claim 13, which is a mouse.

16. Mouse according to anyone of claims 1 to 7 or 9 to 15, which exhibits the following genotype *(SMN$^{F7/\Delta 7}$,* NSE-Cre+) or the following genotype *(SMN$^{F7/F7}$,* NSE-Cre+).

17. Mouse according to anyone of claims 1 to 6 or 8 to 15, which exhibits the following genotype: (SMN $^{F7/\Delta 7}$, HC79-Cre+) or the following genotype *(SMN$^{F7/F7}$,* HC79-Cre+).

18. Non-human mammal according to anyone of claims 1 to 17, which exhibits the following properties:

    1) it expresses a truncated SMN protein in motor neurons, said protein being located to the cytoplasm of cells but not to the nuclear structure of said cells.
    2) it exhibits abnormal coiled bodies and it is essentially devoid of gem structures in motor neurons.
    3) it does not show a substantial loss in neurons.

19. A non-human mammal according to anyone of claims 1 to 18, which exhibits morphological characteristics comprising:

    1) morphological abnormalities in skeletal muscles including denervation of skeletal muscle fibers,
    2) morphological abnormalities in spinal cord, including morphological changes in nuclei of motor neurons, in the absence of significant loss of motor neurons.

20. A population of cells of a non-human mammal according to anyone of claims 1 to 18 which is a primary culture of neurons.

21. A population of cells derived from the non-human mammal according to anyone of claims 1 to 19, wherein the cells are immortalized.

22. A population of cells according to anyone of claims 20 or 21, which is a population of motor neurons, glial cells ependimal cells.

23. Use of a non-human mammal according to anyone of claims 1 to 19, or of a population of cells according to anyone of claims 20 or 22, for the study of neurodegenerative diseases or for the screening of molecules.

24. Use of a non-human mammal according to anyone of claims 1 to 19, or of a population of cells according to anyone of claims 20 to 22, for the study of SMA.

25. Use of a non-human mammal according to anyone of claims 1 to 19 for the study of muscular dystrophy.

26. Process for the comparative analysis of transcripts present in first and second populations of cells of spinal cord, wherein said first population of cells exhibits a homozygous mutation of the *SMN* gene thereby expressing in said first population of cells, a SMN protein which is truncated at its C-terminal end and wherein, said second population of cells of spinal cord exhibits a normal *SMN* gene thereby expressing a normal SMN protein in said second population of cells, said process comprising:

- isolating the RNA transcripts of said first and second populations of cells of spinal cord, to thereby obtain respectively a first and a second populations of transcripts,
- detecting the specific presence or absence, in said first or second population of transcripts, of one of several specific transcripts, thereby detecting differentially expressed transcripts in said one or second population of transcripts.

27. Process according to claim 26, which further comprises the isolation and identification of the RNA transcripts which are specific to only one of the compared first and second populations of transcripts (differentially expressed transcripts).

28. Process according to claims 26 or 27 which further comprises preparation of DNA sequences corresponding to one or several of the differentially expressed transcripts.

29. Process according to anyone of claims 26 to 28 which further comprises the expression of the products encoded by one or several of the differentially expressed transcripts.

30. Process according to anyone of claims 26 to 29, wherein the comparative analysis of the transcripts is performed after the administration of a determined molecule, to the assayed populations of cells.

31. Process for the comparative analysis of the protein expression pattern in a first and a second populations of cells of spinal cord, wherein said first population of cells exhibits a homozygous mutation of the *SMN* gene leading to the expression in said first population of cells, of a SMN protein which is truncated at its C-terminal end and wherein, said second population of cells of spinal cord exhibits a normal *SMN* gene thereby expressing a normal SMN protein in said second population of cells, said process comprising:

- comparing the protein expression pattern of said first and second populations of cells of spinal cord,
- detecting the presence or absence, in said first populations of cells, of one of several specific proteins, of the specific protein(s) thereby identifying differentially expressed proteins.

32. Process according to anyone of claims 26 to 31, wherein populations of cells are assayed at a determined phase of their division or differentiation.

33. Process according to claim 31 or 32 wherein the comparative analysis of the protein expression pattern is carried out after the administration of a determined molecule to the assayed population of cells.

34. Process according to anyone of claims 26 to 33, wherein the first and second populations of cells are obtained from muscle tissue.

35. Process for the evaluation of possible reversion of the neurodegenerative process occurring in SMA disease, which comprises:

- administering to a non-human mammal according to anyone of claims 1 to 19, or to a culture of cells according to anyone of claims 20 to 22, an intact *SMN* gene placed under the control of regulatory sequences enabling the conditional spatial and/or temporal expression of said intact *SMN* gene in said non-human mammal or said culture of cells,
- detecting a change in the phenotype or the restoration of a normal phenotype in said non-human mammal or said culture of cells.

36. Process for the evaluation of possible reversion of the neurodegenerative process occurring in muscular disease, which comprises:

- administering to a non-human mammal according to anyone of claims 1 to 19, or to a culture of cells according to anyone of claims 20 to 22, an intact *SMN* gene placed under the control of regulatory sequences enabling the conditional spatial and/or temporal expression of said intact *SMN* gene in said non-human mammal or said culture of cells,
- detecting a change in the phenotype or the restoration of a normal phenotype in said non-human mammal or said culture of cells.

37. Process according to claim 35 or 36, wherein the intact *SMN* gene is delivered by a recombinant vector selected from viral vectors, retroviral vectors, lentiviral vectors, human or non-human adenovirus vector, canine adenovirus vectors, or a synthetic vector.

38. Process according to claim 35 or 36 or 37, wherein the administration of the *SMN* gene is accompanied by the previous, simultaneous or delayed administration of a determined molecule capable of interacting with or enhancing the activity of the intact *SMN* gene.

39. Process according to anyone of claims 30, 33 or 37, wherein the administered molecule is selected from neurotrophic factors, neuroprotective agents, neuromodulators, neurotransmitter molecules, neuropeptides, the SMN protein, differentially expressed genes or protein products identified in a process according to anyone of claims 26 to 34.

40. Use of a non-human mammal according to anyone of claims 1 to 19 or of a population of cells according to anyone of claims 20 to 22 for the study of neurodegenerative processes.

41. Use of a non-human mammal according to claims 1 to 19 for the study of splicing defect of RNAs in cells.

42. Use according to claim 40 or 41 or process according to anyone of claims 26 to 34 for the analysis of the transcription and/or expression of the dystrophin gene.

43. Use according to claim 40 or 41 or process according to anyone of claims 26 to 34 for the analysis of the transcription and/or expression of gene having large intronic sequences.

44. Process for the evaluation of the activity of a determined molecule on neurodegenerative of neuronal cells or muscular cells populations comprising administering said molecule to a non-human mammal according to anyone of claims 1 to 19, or of a population of cells according to anyone of claims 20 to 22, and detecting the effect of said molecule on the tested cells populations.

45. Process according to claim 44, wherein the tested cells are motor neurons.

FIGURE 1

EP 1 116 790 A1

FIGURE 2

EP 1 116 790 A1

FIGURE 3A

FIGURE 3B

FIGURE 4

FIGURE 5A

FIGURE 5B

FIGURE 6A

FIGURE 6A

FIGURE 6B

FIGURE 7A

FIGURE 7B

**European Patent Office**

# PARTIAL EUROPEAN SEARCH REPORT

Application Number

which under Rule 45 of the European Patent Convention EP 00 40 0100 shall be considered, for the purposes of subsequent proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE BIOSIS [Online] BIOSCIENCES INFORMATION SERVICE, PHILADELPHIA, PA, US; July 1999 (1999-07) TIZIANO FRANCESCO ET AL: "Creation and characterization of transgenic mice expressing Cre recombinase in skeletal muscle and neurons." Database accession no. PREV199900432105 XP002147722 * abstract * & EUROPEAN JOURNAL OF HUMAN GENETICS, vol. 7, no. SUPPL. 1, July 1999 (1999-07), pages 113-114, 31st Annual Meeting of the European Society of Human Genetics;Geneva, Switzerland; May 29-June 1, 1999 ISSN: 1018-4813 --- -/-- | 1-19 | C12N15/12 C07K14/475 C12Q1/68 A61K48/00 A01K67/027 G01N33/68 |

| | | | TECHNICAL FIELDS SEARCHED (Int.Cl.7) |
|---|---|---|---|
| | | | C12N C07K A61K G01N C12Q |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claims 35-39 and 44-45 are directed to a diagnostic method practised on the human/animal body (Article 52(4) EPC), the search has been carried out and based on the alleged effects of the compound/composition.

| Place of search | Date of completion of the search | Examiner |
|---|---|---|
| MUNICH | 19 September 2000 | Julia, P |

EPO FORM 1503 03.82 (P04C07)

**European Patent Office**

# PARTIAL EUROPEAN SEARCH REPORT

**Application Number**

EP 00 40 0100

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | DATABASE BIOSIS [Online] BIOSCIENCES INFORMATION SERVICE, PHILADELPHIA, PA, US; July 1999 (1999-07) FRUGIER TONY ET AL: "Creation and characterization of SMN exon 7-deficient mice." Database accession no. PREV199900416327 XP002147723 * abstract * & EUROPEAN JOURNAL OF HUMAN GENETICS, vol. 7, no. SUPPL. 1, July 1999 (1999-07), page 101 31st Annual Meeting of the European Society of Human Genetics;Geneva, Switzerland; May 29-June 1, 1999 ISSN: 1018-4813 --- | 1-19 | |
| X | EP 0 711 833 A (INST NAT SANTE RECH MED) 15 May 1996 (1996-05-15) | 1-4,6,9, 11-15, 18-20, 22-25,40 | |
| Y | * the whole document, in particular page 10 lines 39-40, page 21 example 13, claims 25 and 38 * --- | 5,7,8, 10,16, 17,21, 26-39, 41-45 | **TECHNICAL FIELDS SEARCHED** (Int.Cl.7) |
| Y | US 5 849 995 A (NASIR JAMAL ET AL) 15 December 1998 (1998-12-15) | 5,7,8, 10,16, 17,21, 26-39, 41-45 | |
| | * the whole document, in particular column 8 lines 12-29, column 9 lines 19-33 and column 10 lines 30-38 * --- -/-- | | |

EPO FORM 1503 03.82 (P04C10)

| | | | European Patent Office | PARTIAL EUROPEAN SEARCH REPORT | Application Number EP 00 40 0100 |

## DOCUMENTS CONSIDERED TO BE RELEVANT

| Category | Citation of document with indication, where appropriate, of relevant passages | Relevant to claim |
|---|---|---|
| A | DATABASE MEDLINE [Online] National Library of Medicine; PMID=9630749, U. GRIESHAMMER ET AL.,: "Muscle-specific cell ablation conditional upon Cre-mediated DNA recombination in transgenic mice leads to massive spinal and cranial motoneuron loss" XP002147724 * abstract * & Dev. Biol. 15-05-1998, Vol. 197(2), pages 234-247 --- | 1,5-19 |
| A | DATABASE BIOSIS [Online] BIOSCIENCES INFORMATION SERVICE, PHILADELPHIA, PA, US; 1997 AKAGI KIWAMU ET AL: "Cre-mediated somatic site-specific recombination in mice." Database accession no. PREV199799565249 XP002147725 * abstract * & NUCLEIC ACIDS RESEARCH, vol. 25, no. 9, 1997, pages 1766-1773, ISSN: 0305-1048 --- | 5,7,8, 10,16,17 |
| A | US 5 882 868 A (FUNANAGE VICKY LINN ET AL) 16 March 1999 (1999-03-16) * the whole document * --- | 26-34 |

-/--

CLASSIFICATION OF THE APPLICATION (Int.Cl.7)

TECHNICAL FIELDS SEARCHED (Int.Cl.7)

European Patent Office

**PARTIAL EUROPEAN SEARCH REPORT**

Application Number

EP 00 40 0100

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | DATABASE BIOSIS [Online] BIOSCIENCES INFORMATION SERVICE, PHILADELPHIA, PA, US; 1998 MINIOU PIERRE ET AL: "Towards the creation of a mouse model for spinal muscular atrophy (SMA) by conditional knock-out of SMN gene." Database accession no. PREV199800383194 XP002147726 * abstract * & EUROPEAN JOURNAL OF HUMAN GENETICS, vol. 6, no. SUPPL. 1, 1998, page 119 30th Annual Meeting of the European Society of Human Genetics;Lisbon, Portugal; May 10-13, 1998 ISSN: 1018-4813 --- | 1-4,6, 12-15 | |
| A | DATABASE BIOSIS [Online] BIOSCIENCES INFORMATION SERVICE, PHILADELPHIA, PA, US; 1997 FRANCIS J W ET AL: "Studies of survival motor neuron (SMN) protein expression in cultured neural cells and the mammalian central nervous system." Database accession no. PREV199799768953 XP002147727 * abstract * & SOCIETY FOR NEUROSCIENCE ABSTRACTS, vol. 23, no. 1-2, 1997, page 550 27th Annual Meeting of the Society for Neuroscience, Part 1;New Orleans, Louisiana, USA; October 25-30, 1997 ISSN: 0190-5295 ----- | 20-25,40 | TECHNICAL FIELDS SEARCHED (Int.Cl.7) |

**ANNEX TO THE EUROPEAN SEARCH REPORT
ON EUROPEAN PATENT APPLICATION NO.**                EP 00 40 0100

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2000

| Patent document cited in search report | | Publication date | Patent family member(s) | | Publication date |
|---|---|---|---|---|---|
| EP 0711833 | A | 15-05-1996 | EP | 0708178 A | 24-04-1996 |
| | | | AU | 702252 B | 18-02-1999 |
| | | | AU | 3436995 A | 02-05-1996 |
| | | | CA | 2160937 A | 20-04-1996 |
| | | | JP | 8228785 A | 10-09-1996 |
| | | | US | 6080577 A | 27-06-2000 |
| US 5849995 | A | 15-12-1998 | CA | 2178022 A | 02-12-1996 |
| US 5882868 | A | 16-03-1999 | NONE | | |

EPO FORM P0459

For more details about this annex : see Official Journal of the European Patent Office, No. 12/82